(19) Europäisches Patentamt / European Patent Office / Office européen des brevets

(11) EP 3 950 687 B1

(12) # FASCICULE DE BREVET EUROPEEN

(45) Date de publication et mention de la délivrance du brevet:
**28.01.2026 Bulletin 2026/05**

(21) Numéro de dépôt: **21306098.1**

(22) Date de dépôt: **06.08.2021**

(51) Classification Internationale des Brevets (IPC):
***C07D 471/04*** *(2006.01)* ***A61K 31/437*** *(2006.01)*
***A61P 17/00*** *(2006.01)*

(52) Classification Coopérative des Brevets (CPC):
**C07D 471/04; A61P 17/00**

(54) **PROCÉDÉ INDUSTRIEL DE SYNTHÈSE DE L'IMIQUIMOD À PARTIR DE LA QUINOLÉINE-2,4-DIOL APPLICABLE À SON UTILISATION PHARMACEUTIQUE**

INDUSTRIELLES VERFAHREN ZUR SYNTHESE VON IMIQUIMOD AUS CHINOLIN-2,4-DIOL FÜR SEINE PHARMAZEUTISCHE ANWENDUNG

INDUSTRIAL METHOD FOR SYNTHESISING IMIQUIMOD FROM QUINOLINE-2,4-DIOL APPLICABLE TO THE PHARMACEUTICAL USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.08.2020 FR 2008366**

(43) Date de publication de la demande:
**09.02.2022 Bulletin 2022/06**

(73) Titulaire: **Generyze**
**39210 Domblans (FR)**

(72) Inventeurs:
- **REFOUVELET, Bernard**
  **25000 Besançon (FR)**
- **ISMAILI, Lhassane**
  **25000 Besançon (FR)**
- **IUTZELER, Anne**
  **39350 Taxenne (FR)**

(74) Mandataire: **Plasseraud IP**
**104 Rue de Richelieu**
**CS92104**
**75080 Paris Cedex 02 (FR)**

(56) Documents cités:
**ES-A1- 2 538 880 FR-A1- 2 921 656**
**US-A- 4 689 338**

- **GERSTER JF ET AL: "Synthesis and structure-activity-relationships of 1H-imidazo[4,5-c] quinolines that induce interferon production", JOURNAL OF MEDICINAL CHEMISTRY, AMERICAN CHEMICAL SOCIETY, US, vol. 48, no. 10, 21 April 2005 (2005-04-21), pages 3481 - 3491, XP008115095, ISSN: 0022-2623, DOI: 10.1021/JM049211V**
- **LINDLEY JAMES: "Tetrahedron report number 163 : Copper assisted nucleophilic substitution of aryl halogen", TETRAHEDRON, vol. 40, no. 9, 1 January 1984 (1984-01-01), AMSTERDAM, NL, pages 1433 - 1456, XP055780951, ISSN: 0040-4020, DOI: 10.1016/S0040-4020(01)91791-0**
- **TOCRIS.COM: "Certificate of Analysis", 22 March 2016 (2016-03-22), pages 1 - 2, XP093074946, Retrieved from the Internet <URL:https://documents.tocris.com/pdfs/tocris_coa/3700_1_coa.pdf?1692363789&_ga=2.247801415.1384133980.1692363792-1546624381.1692363792> [retrieved on 20230818]**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 950 687 B1

## Description

### Domaine technique

La présente invention est relative à un nouveau procédé de synthèse de l'imiquimod, ainsi qu'à son utilisation pour le traitement d'affections cutanées telles que la kératose actinique, le carcinome basocellulaire superficiel, les condylomes acuminés, les dysplasies cervicale et vulvaire.

### Technique antérieure

Plusieurs voies de synthèse de l'imiquimod ou 3-(2-méthylpropyl)-3,5,8-triazatricyclo[7,4,0.02,6]tridéca-1(9),2(6),4,7,10,12-hexaèn-7-amine ou 1-isobutyl-1H-imidazo[4,5-c]quinoléine-4-amine sont décrites dans la littérature. L'étape clef de la synthèse de l'imiquimod correspond à l'introduction de la fonction amine primaire en position 4 de l'imidazo[4,5-c]quinoléine utilisant généralement la chloration, suivi d'une réaction d'aminolyse avec l'ammoniac au départ de la molécule 1-isobutyl-1H-imidazo[4,5-c]quinoline-5-N-oxyde ou de la molécule 4-hydroxy-1-isobutyl-1H-imidazo[4,5-c]quinoline.

Une première voie de synthèse de l'imiquimod est décrite à partir de l'isobutyl-1H-imidazo[4,5-c] quinoléine-N-oxyde dans les brevets EP 0145340, EP 0425306, US 4,689,338 et EP 0145340 (ci-après les « brevets RIKER I, II, III, IV ») (Figure 1). Cet intermédiaire (composé (6) de la Figure 1) est obtenu par oxydation avec l'acide peracétique ou l'eau oxygénée de l'isobutyl-1H-imidazo[4,5-c]quinoléine.

La cyclisation de la 3-amino-4-(isobutylamino)quinoline (composé (4) de la Figure 1) est réalisée classiquement avec un ester orthoformiate. Ce composé est facilement obtenu par réduction du composé (3) tel que décrit à la Figure 1 issu de la 4-chloro-3-nitroquinoléine (composé (2) de la Figure 1) par réaction d'aminolyse avec l'isobutylamine. Ce composé (composé (2) de la Figure 1) est préparé classiquement par chloration du produit de départ (composé (1) de la Figure 1), la 4-hydroxy-3-nitroquinoléine.

L'étape clef de cette synthèse est la formation de la N-oxyde quinoléine (composé (6) de la Figure 1) par l'action de l'eau oxygénée en milieu acide acétique ou acide peracétique, pour permettre l'introduction de l'halogène en position 2 du noyau quinoléine, sous l'action de l'oxychlorure de phosphore.

La dernière étape clef par substitution nucléophile avec l'ammoniaque en solution aqueuse ou dans le méthanol est réalisée sous pression et à haute température (≥ 150°C) avec des rendements assez faibles et un profil d'impuretés non compatible avec un produit de qualité pharmaceutique.

Cette voie de synthèse de l'imiquimod est une voie de synthèse longue, impliquant une synthèse totale en 7 étapes à partir de la 4-hydroxy-3-nitroquinoléine. De plus, les rendements obtenus sont assez faibles et le produit obtenu n'est pas de qualité pharmaceutique.

Une deuxième voie de synthèse de l'imiquimod (Figure 2) développée plus récemment par les laboratoires RIKER également, implique une synthèse totale en 6 étapes à partir de la quinoléine-2,4-diol (EP 0425306, ci-après le brevet RIKER V). Cette méthode est une variante de la méthode précédemment décrite.

L'intérêt de cette voie de synthèse est d'éviter la préparation du dérivé N-oxyde de la quinoléine. Toutefois, là encore la dernière étape implique une réaction d'aminolyse sous pression par l'action de l'ammoniaque à haute température par substitution directe du chlore, sans utilisation de catalyseur. Cette voie de synthèse ne permet pas non plus d'obtenir une substance active de qualité pharmaceutique, compte tenue de l'obtention de nombreux sous-produits de réaction et d'un rendement assez faible.

Une variante de ces 2 voies classiques de synthèse correspond à la synthèse du précurseur de l'imiquimod (la 4-chloro-1H-imidazo[4,5-c]quinoléine) (Figure 3). Cette invention correspond à une variante des 2 méthodes précédentes et implique la formation du noyau imidazole à partir d'un benzaldéhyde halogéné en position 2. La dernière étape clef de cette synthèse implique la formation du noyau quinoléine entre l'atome d'azote de la fonction carboxamide peu nucléophile et l'atome d'halogène du noyau aromatique. Toutefois, l'étape finale clef de la synthèse de l'imiquimod reste inchangée (US 2008/0161573).

Une amélioration intéressante des deux premières voies de synthèse exposées dans les brevets RIKER est décrite par Tarur et al (US 7,678,912 et US 2009/0209764) (Figure 4) et permet d'obtenir une substance active de qualité pharmaceutique. Cette étude concerne un procédé de préparation de l'imiquimod, 4-amino-1-isobutyl-1H-imidazo[4,5-c] quinoléine (composé (8) de la Figure 4). La cyclisation de la 3-amino-4-isobutylaminoquinoléine (composé (1) de la Figure 4) est réalisée par action de l'acide formique pour obtenir la 1-isobutyl-1H-imidazo[4,5-c]quinoléine (composé (2) de la Figure 4). Le dérivé N-oxyde (composé (3) de la Figure 4) est obtenu classiquement par action de l'acide métachloroperbenzoique dont la forme chlorhydrate (composé (4) de la Figure 4) est isolée d'une solution d'acide chlorhydrique éthanolique.

La 4-iodo-1-isobutyl-1H-imidazo[4,5-c]quinoléine (composé (6) de la Figure 4) est préparée par réaction de la 4-chloro-1-isobutyl-1H-imidazo[4,5-c]quinoléine (composé (5) de la Figure 4) avec un halogénure alcalin tel que l'iodure de

sodium. Cette dernière réaction donne des rendements intéressants. Toutefois, cette méthode génère des dérivés iodés qui peuvent être considérés comme de nouvelles impuretés potentiellement toxiques.

D'autres voies de synthèse de l'imiquimod sont connues, mais difficilement industrialisables pour obtenir une substance active de qualité pharmaceutique. Il s'agit de la quatrième voie d'accès dont la première étape implique une substitution nucléophile en position 4 avec le cyanure de sodium. La deuxième étape correspond à l'hydrolyse acide de la fonction nitrile en amide et la troisième étape implique une transposition d'Hofmann en présence d'hypobromite de sodium et conduit à la formation de la fonction amine primaire. Cette quatrième voie de synthèse de l'imiquimod est décrite à partir de l'isobutyl-1H-imidazo[4,5-c]quinoléine-N-oxyde obtenu classiquement par oxydation avec l'acide peracétique de l'isobutyl-1H-imdazo[4,5-c]quinoléine (WO 2004/011462) (Figure 5).

Une cinquième voie d'accès à l'imiquimod (Figure 6) implique un couplage de Suzuki entre un dérivé du pinacol 2-(aminophényl)boronate (composé (I) de la Figure 6) et un imidazole substitué (composé (II) de la Figure 6). La fonction amine primaire étant déjà présente sur l'imidazole de départ (EP 2009002).

Une sixième voie de synthèse de l'imiquimod (Figure 7) implique la condensation d'une 4-halo-1H-imidazo[4,5-c]quinoléine (composé (I) de la Figure 7) avec le formamide (composé (II) de la Figure 7) pour obtenir les composées non isolés (composé (III) de la Figure 7), dont l'hydrolyse conduit aux composés (IV) de la Figure 7, avec $R_1$ = H et $R_2$ = isobutyle pour l'imiquimod. L'intérêt de cette méthode d'introduction de la fonction amine en position 2 du noyau quinoléine est l'absence d'utilisation de la réaction d'aminolyse sous pression en présence d'ammoniaque pour l'obtention de l'imiquimod (WO 2006/100226). Toutefois, cette méthode semble difficilement industrialisable avec des intermédiaires de synthèses non isolés et identifiées.

La dernière voie de synthèse décrite de l'imiquimod correspond à une synthèse totale en 6 étapes à partir de la 3-nitroquinoléine-2,4-diol, sans utilisation de dérivés halogénés (WO 94/17043),(Figure 8). L'intérêt de cette synthèse est l'utilisation de la fonction acide sulfonique comme groupe partant au lieu d'un atome d'halogène (principalement l'atome de chlore) et de l'introduction de la fonction amine en position 2 dès la troisième étape de la synthèse sous forme protégée, mais là encore le procédé est difficilement industrialisable.

Les documents J. Med. Chem. 2005, 48, 3481-3491 et ES 2 538 880 décrivent également des procédés de synthèse de l'imiquimod à partir d'un intermédiaire chloré (4-chloro-1-isobutyl-1H-imidazo[4,5-c]quinoléine). Toutefois, et comme pour le procédé décrit dans US 4, 689, 338, l'étape d'aminolyse se fait sans catalyseur métallique et sous pression ou bien par chauffage dans un solvant comme le DMSO en présence d'un sel d'ammonium ($NH_4Cl$) en milieu basique (KOH) pour générer l'ammoniac in situ.

Ainsi, bien que de très nombreux procédés de synthèse de l'imiquimod soient décrits dans l'état de la technique, la majorité ne permet pas d'obtenir un produit de qualité pharmaceutique avec un rendement élevé. Lorsqu'ils le peuvent, ces procédés sont difficilement industrialisables, longs et très coûteux.

Il est ainsi nécessaire de mettre au point un procédé de synthèse de l'imiquimod offrant un haut rendement, permettant l'obtention de produit de qualité pharmaceutique, facilement industrialisable et rapide.

## Résumé

Par ce nouveau procédé, les inventeurs proposent une amélioration importante de la préparation par synthèse totale de l'imiquimod à partir de la quinoléine-2,4-diol permettant d'obtenir une substance active de qualité pharmaceutique.

Cette méthode de synthèse en 6 étapes à partir de la quinoléine-2,4-diol comprend deux étapes originales de substitutions nucléophiles (étape 3 et 6 de la Figure 9), par rapport à l'art antérieur. La dernière étape est une étape clef de la synthèse, qui utilise un catalyseur à base de cuivre pour la réalisation de l'étape de substitution nucléophile aromatique par aminolyse.

Il est ainsi proposé, selon un **premier** aspect, un procédé de synthèse de l'imiquimod comprenant une étape de substitution nucléophile aromatique par aminolyse réalisée en présence d'au moins un catalyseur métallique choisi parmi les sels de cuivre, les oxydes cuivriques, les oxydes cuivreux et leurs mélanges.

Le procédé selon l'invention permet avantageusement de générer une substance active, l'imiquimod, de haute pureté, utilisable comme molécule de qualité pharmaceutique répondant aux exigences de la Pharmacopée Européenne et des recommandations guidelines ICH Q3A, Q3C et Q3D.

De plus, le procédé de synthèse selon la présente invention est rapide (maximum de 7 heures), facilement industrialisable et offre un haut rendement.

Par ailleurs, les propriétés antivirales et antitumorales de l'imiquimod offrent des voies thérapeutiques pour le traitement de pathologies en dermatologie et gynécologie.

## Brève description des dessins

D'autres caractéristiques, détails et avantages apparaîtront à la lecture de la description détaillée ci-après, et à

l'analyse des dessins annexés, sur lesquels :

**Fig. 1**
[Fig. 1] montre une première voie de synthèse de l'état de la technique telle que décrite dans les brevets RIKER I, II, III et IV (EP 0145340, EP 0425306, US 4,689,338 et EP 0145340).
**Fig. 2**
[Fig. 2] montre une seconde voie de synthèse de l'état de la technique telle que décrite dans le brevet RIKER V (EP 0425306).
**Fig. 3**
[Fig. 3] montre une voie de synthèse alternative aux brevets RIKER telle que décrite dans le brevet US 2008/0161573.
**Fig. 4**
[Fig. 4] montre une troisième voie de synthèse de l'état de la technique telle que décrite dans les brevets US 7,678,912 et US 2009/0209764.
**Fig. 5**
[Fig. 5] montre une quatrième voie de synthèse de l'imiquimod telle que décrite dans l'état de la technique (WO 2004/011462).
**Fig. 6**
[Fig. 6] montre une cinquième voie de synthèse telle que décrite dans l'état de la technique (EP 2009002).
**Fig. 7**
[Fig. 7] montre une sixième voie de synthèse telle que décrite dans l'état de la technique (WO 2006/100226).
**Fig. 8**
[Fig. 8] montre une septième voie de synthèse telle que décrite dans l'état de la technique (WO 94/17043).
**Fig. 9**
[Fig. 9] montre les différentes étapes (6) du procédé selon l'invention.
**Fig. 10**
[Fig. 10] montre l'ensemble des impuretés de synthèse de l'imiquimod.
**Fig. 11**
[Fig. 11] montre l'ensemble des produits de dégradation par dégradation thermique, oxydation ou photo-oxydation.
**Fig. 12**
[Fig.12] montre le chromatogramme de l'imiquimod obtenu selon le procédé selon l'invention (lot IMI01).
**Fig. 13**
[Fig.13] montre le chromatogramme de l'imiquimod obtenu selon les procédés de l'état de la technique (lot IMI02).
**Fig. 14**
[Fig.14] montre le chromatogramme du blanc (solution de dilution).
**Fig. 15**
[Fig.15] montre le chromatogramme du témoin de référence (imiquimod à 0,1%, de TCI Chemicals).

**Description détaillée**

Procédé de synthèse de l'imiquimod

Selon un **premier aspect,** la présente invention concerne un procédé de synthèse d'un composé de formule (VII)

[Chem 1]

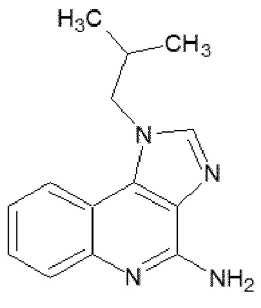

(VII)

caractérisé en ce qu'il comprend une étape de substitution nucléophile aromatique par aminolyse du composé de formule

(VI)

[Chem 2]

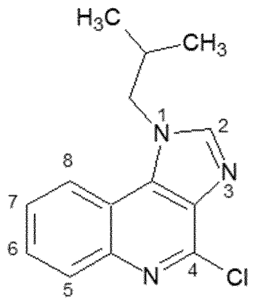

(VI)

,

ladite étape de substitution nucléophile aromatique étant réalisée en présence d'au moins un catalyseur métallique.

La substitution nucléophile aromatique par aminolyse s'entend du déplacement du groupe partant, le chlore en position 4, par une amine, préférentiellement en présence d'ammoniaque.

Selon un mode de réalisation, le catalyseur métallique est choisi parmi les sels de cuivre, les oxydes cuivriques, les oxydes cuivreux et leurs mélanges.

Selon un mode de réalisation, le catalyseur métallique est choisi parmi les sels de cuivre, préférentiellement les sels d'halogénures de cuivre. Typiquement, les sels d'halogénure de cuivre seront choisis parmi le bromure de cuivre, le chlorure de cuivre, l'iodure de cuivre et leurs mélanges. Préférentiellement le catalyseur métallique est le chlorure de cuivre.

Avantageusement, la présence de cuivre permet de réduire le temps et la pression de la réaction, et par conséquent, permet de diminuer le taux des impuretés organiques dans la substance active, l'imiquimod.

De plus et avantageusement, le cuivre est un métal de faible toxicité (classe 3), facilement éliminable par recristallisation de la molécule.

Selon un mode de réalisation, le catalyseur métallique est présent en une teneur allant de 1% à 5% en poids par rapport au poids total des réactifs.

Selon un mode de réalisation, l'étape de substitution nucléophile aromatique par aminolyse est réalisée en présence d'ammoniaque.

Typiquement, pourront être choisis l'ammoniaque en solution aqueuse ou l'ammoniaque en solution méthanolique, préférentiellement une solution méthanolique d'ammoniaque dont la concentration est comprise entre 10 et 15% et plus préférentiellement dont la concentration est égale à environ 12%.

Selon un mode de réalisation, la solution d'ammoniaque est présente en une teneur comprise entre 80 et 95% en poids par rapport au poids total des réactifs.

Selon un mode de réalisation, l'étape de substitution nucléophile aromatique par aminolyse est réalisée sous pression, la pression étant comprise entre 10 et 15 bars.

Selon un mode de réalisation, l'étape de substitution nucléophile aromatique par aminolyse est réalisée à une température inférieure ou égale à 160°C, de préférence comprise entre 120°C et 160°C. Préférentiellement, la température de la réaction est de 145°C.

Selon un mode de réalisation, le procédé selon l'invention comprend en outre une étape de substitution nucléophile du composé de formule (III)

[Chem 3]

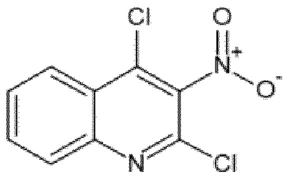

composé (III)

pour l'obtention d'un composé de formule (IV),

[Chem 4]

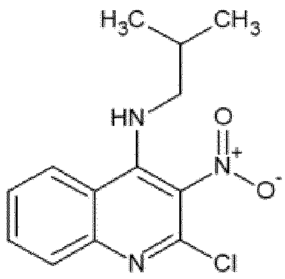

composé (IV)

,

ladite étape de substitution nucléophile étant réalisée en présence d'isobutylamine, d'au moins un catalyseur tel que la triéthylamine et d'au moins un solvant polaire aprotique.

Le composé de formule (III) par réaction de substitution nucléophile en position 4 avec l'isobutylamine (ou 2-méthylpropylamine) en présence d'un catalyseur tel que la triéthylamine conduit au composé de formule (IV).

Typiquement, le catalyseur sera choisi parmi, la triéthylamine, la pyridine ou la pipéridine.

Préférentiellement, le catalyseur est la triéthylamine.

Typiquement, le solvant polaire aprotique est choisi parmi le tétrahydrofurane (THF), le diméthylformamide (DMF), le dichlorométhane (DCM).

Préférentiellement, le solvant polaire aprotique est le dichlorométhane.

Selon un mode de réalisation, la température de cette étape de substitution nucléophile est comprise entre 40°C et 50°C. Elle est de préférence de 44°C.

Selon un mode de réalisation, le temps de réaction de cette étape de substitution nucléophile est compris entre 20 et 40 minutes, de préférence 30 minutes.

Cette étape de substitution nucléophile apparait régiosélective en position 4 du noyau quinoléine sans formation de l'isomère en position 2, grâce au contrôle de la température de la réaction en favorisant le produit cinétique et par l'utilisation d'un solvant polaire aprotique en présence de triéthylamine.

Les deux étapes de substitution nucléophile précédemment décrites du procédé selon l'invention, sont d'une part nouvelles et originales par rapport aux procédés décrits dans l'état de la technique, et permettent surtout et avantageusement, de générer une substance active, l'imiquimod, de haute pureté, utilisable comme molécule de qualité pharmaceutique répondant aux exigences de la Pharmacopée Européenne et des recommandations guidelines ICH Q3A, Q3C et Q3D.

Selon un mode de réalisation, la première étape du procédé selon l'invention est une étape de nitration de la quinoline-2,4-diol, de formule (I)

[Chem 5]

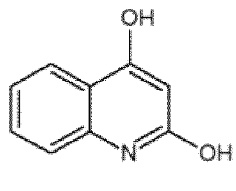

(I)

,

pour l'obtention d'un composé de formule (II)

[Chem 6]

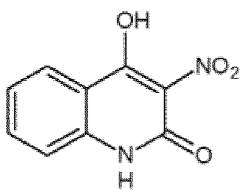

(II)

.

Le procédé selon l'invention utilise comme matériel de départ ou « starting material », la quinoline-2,4-diol (CAS : 86-95-3).

Selon un mode de réalisation, l'étape de nitration est réalisée en présence d'acide nitrique ($HNO_3$).

Selon un mode de réalisation, la température de cette première étape est comprise entre 70°C et 80°C, de préférence, la température est de 75°C.

Selon un mode de réalisation, la seconde étape du procédé selon l'invention est une étape de de chloration en position 2 et 4 du composé de formule (II) pour l'obtention d'un composé de formule (III).

Typiquement, l'étape de chloration est réalisée en présence d'oxychlorure de phosphore ($POCl_3$).

Selon un mode de réalisation, la troisième étape du procédé correspond à l'étape de substitution nucléophile précédemment décrite, du composé de formule (III)

[Chem 3]

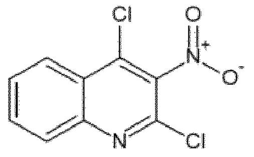

composé (III)

pour l'obtention d'un composé de formule (IV),

[Chem 4]

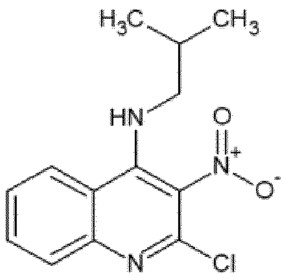

composé (IV)

Selon un mode de réalisation, la quatrième étape du procédé selon l'invention est une étape de réduction par hydrogénation catalytique du composé de formule (IV) pour l'obtention d'un composé de formule (V)

[Chem 7]

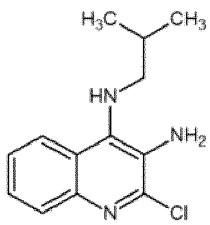

composé (V)

Selon un mode de réalisation, l'hydrogénation catalytique est réalisée en présence d'un métal. Typiquement, le métal est choisi parmi le platine (Pt), le palladium (Pd), le nickel (Ni). Préférentiellement, le métal est le palladium sur charbon (Pd/C).

Selon un mode de réalisation, cette étape est réalisée sous pression d'hydrogène pendant 3 à 6 heures, préférentiellement 5 heures.

Selon un mode de réalisation, la cinquième étape du procédé selon l'invention est une étape de cyclisation à partir du composé de formule (V) pour l'obtention d'un composé de formule (VI)

[Chem 8]

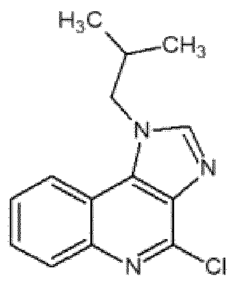

composé (VI)

,

ladite cyclisation étant réalisée en présence de triéthylorthoformiate.

Cette étape de cyclisation permet la formation du noyau imidazole.

Selon un mode de réalisation, cette cinquième étape est réalisée en présence d'un solvant tel que le toluène, par chauffage à une température comprise entre 100°C et 120°C, de préférence, à une température d'environ 110°C pendant une durée comprise entre 1 heure et 3 heures, préférentiellement 2 heures.

Selon un mode de réalisation, la sixième étape du procédé est l'étape de substitution nucléophile aromatique par aminolyse, telle que précédemment décrite, du composé de formule (VI) pour l'obtention du composé de formule (VII).

Cette sixième étape permet l'obtention du composé de formule (VII), l'imiquimod.

Imiquimod

Ainsi, et selon un **deuxième aspect,** la présente invention concerne l'imiquimod susceptible d'être obtenu par le procédé selon l'invention.

L'imiquimod ou 1-isobutyl-1H-imidazo[4,5-c]quinoléine-4-amine ($C_{14}H_{16}N_4$ ; CAS : 99011-02-6) est le composé de formule (VII)

[Chem 1]

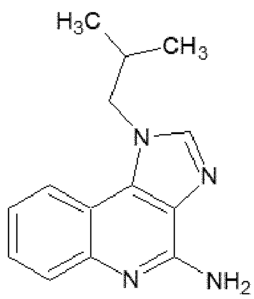

(VII)

.

Toute technique connue de l'homme du métier pourra être utilisée pour caractériser l'imiquimod.

A titre illustratif, l'imiquimod pourra être caractérisé et identifié par spectrophotométrie infrarouge FT-IR selon la méthode de la Pharmacopée Européenne. 2.2.24 (entre 600 et 4000 $cm^{-1}$) et/ou par la réalisation des spectres RMN $^1H$ et $^{13}C$ dans le DMSOd6 selon la méthode de la Pharmacopée Européenne. 2.2.33.

Pureté de l'imiquimod obtenu

Les impuretés pouvant être obtenues suite à la mise en œuvre du procédé selon l'invention sont les impuretés de synthèse (telles que décrites à la Figure 10) et les produits de dégradation par dégradation thermique, oxydation ou photo-oxydation tels que décrits à la Figure 11.

Toute technique connue de l'homme du métier pour mesurer la pureté du composé obtenu pourra être utilisée. Typiquement, la méthode CLHP/UV pourra être utilisée (Hussain S, Shaikh T, and Farooqui M, Development and Validation of Liquid Chromatography Method for the Determination and Quantification of Impurities in Imiquimod. Journal of Pharmaceutical Research International. 13, 1 (Sep. 2016), 1-9).
Avantageusement, l'imiquimod obtenu selon le procédé de l'invention présente, moins de 0,2% d'impuretés en poids, (et préférentiellement moins de 0,1% d'impuretés en poids, par rapport au poids total d'imiquimod obtenu.
Ainsi, le procédé selon l'invention permet d'obtenir l'imiquimod d'une pureté supérieure ou égale à 99%.
Ainsi, la présente invention concerne également l'imiquimod de formule (VII)

[Chem 1]

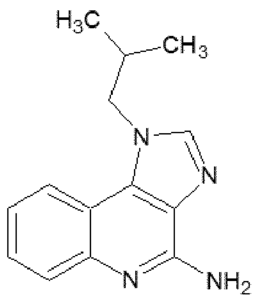

(VII)

d'une pureté supérieure ou égale à 95%, préférentiellement supérieure ou égale à 96%, préférentiellement supérieure ou égale à 97%, préférentiellement supérieure ou égale à 98% et préférentiellement supérieure ou égale à 99%.

Rendement

L'imiquimod est obtenu par le procédé de synthèse selon l'invention avec un rendement élevé, généralement supérieur à 80%, préférentiellement supérieur ou égal à 85%.

Prévention et traitement des affections cutanées

L'imiquimod est un modificateur de la réponse immunitaire. Son mécanisme d'action repose essentiellement sur son rôle en tant que ligand des "Toll-Like receptor (TLRs) 7 et 8". Cela lui confère ses propriétés antivirales en générant une synthèse accrue d'interféron alpha, des cytokines pro-inflammatoires dont l'interleukine 6, des chimiokines et des facteurs de nécrose tumorale (Ambach A, Bonnekoh B, Nguyen M, et al. Imiquimod, a Toll-like Receptor-7 Agonist, Induces Perforin in Cytotoxic T Lymphocytes in Vitro. Mol Immunol 2004, 40, 1307-1314).
L'imiquimod agit également sur les voies des caspases favorisant l'apoptose cellulaire, ce qui lui confère des propriétés anti-tumorales.
Ainsi, et selon un mode de réalisation, l'invention concerne l'utilisation d'imiquimod obtenu selon le procédé selon l'invention, comme médicament.
Elle concerne également l'imiquimod de formule (VII)

[Chem 1]

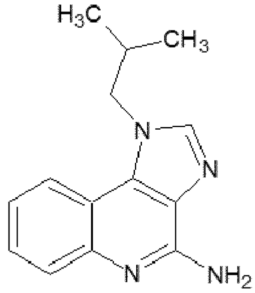

(VII)

d'une pureté supérieure ou égale à 95%, préférentiellement supérieure ou égale à 96%, préférentiellement supérieure ou égale à 97%, préférentiellement supérieure ou égale à 98% et préférentiellement supérieure ou égale à 99%, pour son utilisation comme médicament.

L'imiquimod est le principe actif d'ALDARA®, et est utilisé en dermatologie pour le traitement topique des pathologies suivantes :

- Carcinomes basocellulaires superficiels de l'adulte ;
- Kératoses actiniques cliniquement typiques, non hypertrophiques, non hyperkératosiques du visage ou du cuir chevelu chez l'adulte immunocompétent.

Il est également utilisé en gynécologie et a montré son efficacité dans le traitement des lésions induites par le papillomavirus humain telles les verrues génitales et péri anales externes (condylomes acuminés) de l'adulte et les dysplasies cervicale et vulvaire, en stimulant l'immunité du sujet infecté.

Ainsi, et selon un **troisième aspect,** la présente invention concerne l'imiquimod pour son utilisation pour le traitement des affections cutanées telles que la kératose actinique, le carcinome basocellulaire superficiel, les condylomes acuminés, les dysplasies cervicale et vulvaire.

Selon un mode de réalisation, la présente invention concerne l'imiquimod obtenue par le procédé selon l'invention, pour son utilisation pour le traitement des affections cutanées telles que la kératose actinique, le carcinome basocellulaire superficiel, les condylomes acuminés, les dysplasies cervicale et vulvaire.

Selon un mode de réalisation, l'invention concerne l'imiquimod de formule (VII)

[Chem 1]

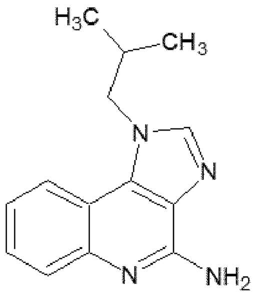

(VII)

d'une pureté supérieure ou égale à 95%, préférentiellement supérieure ou égale à 96%, préférentiellement supérieure ou égale à 97%, préférentiellement supérieure ou égale à 98% et préférentiellement supérieure ou égale à 99%, pour son utilisation pour le traitement des affections cutanées telles que la kératose actinique, le carcinome basocellulaire superficiel, les condylomes acuminés, les dysplasies cervicale et vulvaire.

Les termes « traitement » ou « méthode de traitement », ne sont pas des termes absolus et, lorsqu'ils sont appliqués à des affections cutanées telles que la kératose actinique, le carcinome basocellulaire superficiel, les condylomes acuminés, les dysplasies cervicale et vulvaire, ils désignent une procédure ou un plan d'action conçu, même avec une probabilité de succès faible mais devant induire un effet bénéfique global tel que le retard d'apparition de la pathologie, ou la diminution de la gravité d'un ou plusieurs symptômes ou la stabilisation de la pathologie.

Selon un mode de réalisation, l'imiquimod pourra être administré sous forme d'une composition pharmaceutique comprenant, outre l'imiquimod, un excipient pharmaceutiquement acceptable.

On entend par « pharmaceutiquement acceptable » une substance qui n'est pas biologiquement ou autrement indésirable, c'est-à-dire qui peut être incorporée dans une composition pharmaceutique administrée à un patient sans causer d'effets biologiques indésirables ou sans interagir de manière délétère avec l'un des autres composants de la composition dans laquelle elle est contenue, par exemple en inhibant ou diminuant les propriétés antivirales et anti tumorales de l'imiquimod.

Typiquement, l'excipient pharmaceutiquement acceptable pourra être choisi parmi un diluant, un désintégrant, un liant, un agent de glissement, un agent lubrifiant, un agent mouillant, un agent tampon, un agent de suspension, un adjuvant, un émulsifiant, un absorbant, un conservateur, un agent de surface, un agent édulcorant, un antioxydant, ou un mélange de ceux-ci. Ces excipients sont par exemple décrits dans « The Science and Practice of Pharmacy 1995, edited by E. W. Martin, Mack Publishing Company, 19th edition, Easton, Pa ».

La quantité d'imiquimod dans les compositions peut varier de manière à administrer une quantité efficace d'imiquimod pour obtenir la réponse thérapeutique souhaitée pour un patient particulier.

Par « quantité efficace » ou « quantité thérapeutiquement efficace » d'un composé, on entend une quantité non toxique mais suffisante du composé pour fournir l'effet souhaité.

Typiquement, la quantité administrée ou dose, dépend de l'activité de l'imiquimod, de la voie d'administration, de la gravité de la pathologie, ainsi que de l'état de santé et des antécédents médicaux du patient traité, de divers facteurs tels que le poids corporel, du régime alimentaire, de l'éventuelle combinaison avec d'autres agents thérapeutiques. Il est toutefois de la compétence de l'homme du métier de déterminer le dosage adéquat et d'initier le traitement à un dosage inférieur à celui requis pour obtenir l'effet thérapeutique souhaité et d'augmenter progressivement la dose jusqu'à ce que l'effet souhaité soit obtenu.

Les compositions pharmaceutiques selon la présente invention pourront être administrées par voie topique. Typiquement, les compositions pharmaceutiques pourront se présenter sous la forme physique d'une crème, d'une pommade, d'un gel, d'une solution.

Les méthodes de fabrication des formulations pharmaceutiques pour une administration par voie topique sont connues de l'homme de l'art et peuvent être utilisées pour préparer les présentes compositions.

## Exemples

Dans les exemples qui suivent, les méthodes ci-après décrites ont été utilisées.

*Identification de l'imiquimod (composé VII tel que décrit à la Figure 9) selon la pharmacopée européenne en vigueur*

L'imiquimod obtenu est contrôlé selon une monographie interne développée en accord avec les recommandations et les méthodes générales décrites à la Pharmacopée Européenne (Ph. Eur.) en vigueur.

Identification par spectrophotométrie infrarouge FT-IR selon la méthode de la Ph. Eur. 2.2.24 (entre 600 et 4000 $cm^{-1}$)

Spectre infrarouge : 2853 $cm^{-1}$, 1667 $cm^{-1}$, 1605 $cm^{-1}$, 1525 $cm^{-1}$, 1480 $cm^{-1}$, 1438 $cm^{-1}$, 1411 $cm^{-1}$, 1324 $cm^{-1}$, 1266 $cm^{-1}$, 1186 $cm^{-1}$, 1166 $cm^{-1}$, 1147 $cm^{-1}$, 1116 $cm^{-1}$, 1027 $cm^{-1}$, 866 $cm^{-1}$, 764 $cm^{-1}$, 754 $cm^{-1}$, 666 $cm^{-1}$, 613 $cm^{-1}$.

Les spectres RMN $^{1}H$ et $^{13}C$ du composé de formule (VII) sont réalisés dans le DMSOd6 selon la méthode de la Ph. Eur. 2.2.33.

Spectre RMN $^{1}H$ à 300 MHz dans DMSOd6 : 0,92 ppm, (d, 6H, $CH_3$) ; 2,21 ppm, (m, 1H, CH) ; 4,41 ppm, (d, 2H, N-$CH_2$) ; 6,59 ppm, (s, 2H, $NH_2$) ; 7,29 ppm, (m, 1H, CH en 6) ; 7,46 ppm, (m, 1H, CH en 7) ; 7,63 ppm, (m, 1H, CH en 8) ; 8,01 ppm (d, 1H, CH en 9) ; 8,18 (s, 1H CH en 5).

Spectre RMN $^{13}C$ à 300 MHz dans DMSOd6 : 20,1 ($CH_3$) ; 30,6 (CH) ; 60,5 ($CH_3$) ; 113,3 (Ar en 4) ; 118,9 (Ar en 3) ; 121,7 (Ar en 5) ; 130,4 (Ar en 7) ; 136,2 (Ar en 6) ; 137,1 (Ar en 8a) ; 138,7 (Ar en 8) ; 145,9 (Ar en 9) ; 148,2 (Ar en 2) ; 148,69 (C4) ; 161,85 (NCO) ; 164,99 (C2) ; 168,57 (OCO).

Les déplacements chimiques sont résumés dans les tables suivantes :

[Tableau1]

| Tableau 1 : RMN $^{1}H$ (imiquimod - composé de formule (VII)) | | | |
|---|---|---|---|
| **δ ppm** | **multiplicité** | **intégration** | **attribution** |
| 8,18 | s | 1H | CH en 5 |
| 8,01 | d | 1H | CH en 9 (imidazole) |
| 7,63 | d | 1H | CH en 8 |
| 7,46 | m | 1H | CH en 7 |
| 7,29 | m | 1H | CH en 6 |
| 6,59 | s | 2H | $NH_2$ |
| 4,41 | d | 2H | N-$CH_2$ |
| 2,21 | m | 1H | CH |
| 0,92 | d | 6H | 2 x $CH_3$ |

[Tableau 2]

| Tableau 2 : RMN $^{13}C$ (imiquimod- composé de formule(VII)) | |
|---|---|
| **δ ppm** | **attribution** |
| 148,2 | C en 2 |
| 137,1 | C en 8a |
| 145,9 | C en 9 |
| 138,7 | C en 4 |
| 136,2 | C en 6 |
| 130,4 | C en 7 |
| 124,5 | C en 8 |
| 118,9 | C en 3 |
| 121,7 | C en 5 |
| 133,3 | C en 4a |
| 60,5 | $CH_2$ |
| 30,6 | CH |
| 20,1 | 2 x $CH_3$ |

*Identification du 3-nitroquinolin-2,4-diol (composé de formule (II) tel que décrit à la Figure 9)*

Le 3-nitroquinolin-2,4-diol obtenu est identifié par spectrophotométrie infrarouge FT-IR selon la méthode de la Pharmacopée Européenne (Ph. Eur.) 2.2.24 (entre 600 et 4000 $cm^{-1}$).
Les spectres RMN $^{1}H$ et $^{13}C$ du composé de formule (II) sont réalisés dans le DMSOd6 selon la méthode de la Ph. Eur. 2.2.33.
Les déplacements chimiques sont résumés dans les tables suivantes :

[Tableau 3]

| Tableau 3 : RMN $^{1}H$ (composé de formule (II)) | | | |
|---|---|---|---|
| δ ppm | multiplicité | intégration | attribution |
| 7,32 | m | 2H | H en 7 ; 5 |
| 7,65 | dd | 1H | H en 6 |
| 8,03 | d | 1H | H en 8 |
| 11,97 | s | 1H | OH |

[Tableau 4]

| Tableau 4 : RMN $^{13}C$ (composé de formule (II)) | |
|---|---|
| **δ ppm** | **attribution** |
| 156,9 | C en 2 |
| 156,3 | C en 4 |
| 138,6 | C en 8a |
| 133,6 | C en 7 |
| 124,9 | C en 5 |
| 122,8 | C en 8 |
| 116,3 | C en 4a |

(suite)

| Tableau 4 : RMN $^{13}C$ (composé de formule (II)) | |
|---|---|
| **δ ppm** | **attribution** |
| 114,7 | C en 3 |

*Identification du composé de formule (III) tel que décrit à la Figure 9*

Le composé de formule (III) obtenu est identifié par spectrophotométrie infrarouge FT-IR selon la méthode de la Ph. Eur. 2.2.24 (entre 600 et 4000 $cm^{-1}$).

Les spectres RMN $^{1}H$ et $^{13}C$ du composé de formule (III) sont réalisés dans le DMSOd6 selon la méthode de la Ph. Eur. 2.2.33.

Les déplacements chimiques sont résumés dans les tables suivantes :

[Tableau 5]

| Tableau 5 : RMN $^{1}H$ (composé de formule (III)) | | | |
|---|---|---|---|
| **δ ppm** | **multiplicité** | **intégration** | **attribution** |
| 8,28 | m | 1H | H en 8 |
| 8,12 | m | 1H | H en 6 |
| 7,94 | m | 1H | H en 5 |
| 7,83 | m | 1H | H en 7 |

[Tableau 6]

| Tableau 6 : RMN $^{13}C$ (composé de formule (III)) | |
|---|---|
| **δ ppm** | **attribution** |
| 133,4 | C en 2 et 4 |
| 129,7 | C en 8a et 7 |
| 129,4 | C en 5 et 8 |
| 125,2 | C en 4 et 3 |

*Identification du composé de formule (IV) tel que décrit à la Figure 9*

Le composé de formule (IV) obtenu est identifié par spectrophotométrie infrarouge FT-IR selon la méthode de la Ph. Eur. 2 .2.24 (entre 600 et 4000 $cm^{-1}$).

Les spectres RMN $^{1}H$ et $^{13}C$ du composé de formule (IV) sont réalisés dans le DMSOd6 selon la méthode de la Ph. Eur. 2.2.33.

Les déplacements chimiques sont résumés dans les tables suivantes :

[Tableau 7]

| Tableau 7 : RMN $^{1}H$ (composé de formule (IV)) | | | |
|---|---|---|---|
| **δ ppm** | **multiplicité** | **intégration** | **attribution** |
| 7,94 | m | 2H | H en 7 et 5 |
| 7,75 | m | 1H | H en 6 |
| 7,54 | m | 1H | H en 8 |
| 6,20 | s | 1H | NH |
| 3,32 | m | 2H | $-NH-CH_2-$ |

(suite)

| Tableau 7 : RMN ¹H (composé de formule (IV)) | | | |
|---|---|---|---|
| **δ ppm** | **multiplicité** | **intégration** | **attribution** |
| 1,96 | m | 1H | 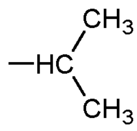 |
| 1,03 | m | 6H | 2 x $CH_3$ |

[Tableau 8]

| Tableau 8 : RMN ¹³C (composé de formule (IV)) | |
|---|---|
| **δ ppm** | **attribution** |
| 132,1 | C en 4 |
| 129,7 | C en 6 |
| 126,4 | C en 7 |
| 122,3 | C en 8 |
| 53,4 | $-NH-CH_2-$ |
| 29,7 | 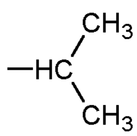 |
| 20,0 | 2 x $CH_3$ |

*Identification du composé de formule (V) tel que décrit à la Figure 9*

Le composé de formule (V) obtenu est identifié par spectrophotométrie infrarouge FT-IR selon la méthode de la Ph. Eur. 2.2.24 (entre 600 et 4000 $cm^{-1}$).

Les spectres RMN $^1H$ et $^{13}C$ du composé de formule (V) sont réalisés dans le DMSOd6 selon la méthode de la Ph. Eur. 2.2.33.

Les déplacements chimiques sont résumés dans les tables suivantes :

[Tableau 9]

| Tableau 9 : RMN ¹H (composé de formule (V)) | | | |
|---|---|---|---|
| **δ ppm** | **multiplicité** | **intégration** | **attribution** |
| 8,02 | m | 1H | H en 8 |
| 7,66 | m | 1H | H en 6 |
| 7,40 | m | 2H | H en 5 et 7 |
| 5,33 | s | 1H | NH |
| 5,07 | s | 2H | $NH_2$ |
| 3,08 | m | 2H | $-NH-CH_2-$ |
| 1,77 | m | 1H | 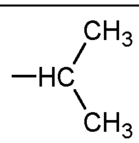 |
| 0,87 | m | 6H | 2 x $CH_3$ |

[Tableau 10]

| Tableau 10 : RMN $^{13}C$ (composé de formule (V)) | |
|---|---|
| **δ ppm** | **attribution** |
| 132,1 | C en 4 |
| 128,7 | C en 6 |
| 126,5 | C en 7 |
| 122,3 | C en 8 |
| 53,4 | $CH_2$ |
| 29.7 | CH |
| 20,1 | 2 x $CH_3$ |

*<u>Identification du composé de formule (VI) tel que décrit à la Figure 9</u>*

Le composé de formule (VI) obtenu est identifié par spectrophotométrie infrarouge FT-IR selon la méthode de la Ph. Eur. 2.2.24 (entre 600 et 4000 $cm^{-1}$).

Les spectres RMN $^{1}H$ et $^{13}C$ du composé de formule (VI) sont réalisés dans le DMSOd6 selon la méthode de la Ph. Eur. 2.2.33.

Les déplacements chimiques sont résumés dans les tables suivantes :

[Tableau 11]

| Tableau 11 : RMN $^{1}H$ (composé de formule (VI)) | | | |
|---|---|---|---|
| δ ppm | multiplicité | intégration | attribution |
| 8,49 | s | 1H | H en 6 |
| 8,34 | m | 1H | H en 8 |
| 8,10 | m | 1H | H en 2 |
| 7,77 | m | 2H | H en 5 et 7 |
| 4,55 | d | 2H | -NH-$CH_2$- |
| 2,22 | m | 1H | 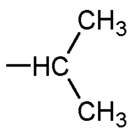 |
| 0,94 | d | 6H | 2 x $CH_3$ |

[Tableau 12]

| Tableau 12 : RMN $^{13}C$ (composé de formule (VI)) | |
|---|---|
| **δ ppm** | **attribution** |
| 146,5 | C en 2 |
| 143,6 | C en 8a |
| 143,4 | C en 9 |
| 134,4 | C en 4 et 4a |
| 129,6 | C en 6 |
| 128,5 | C en 7 |
| 127,6 | C en 8 |
| 121,7 | C en 3 |

(suite)

| Tableau 12 : RMN $^{13}C$ (composé de formule (VI)) | |
|---|---|
| **δ ppm** | **attribution** |
| 117,9 | C en 5 |
| 54,1 | $CH_2$ |
| 28,8 | CH |
| 19,7 | 2 x $CH_3$ |

*Méthode CLHP/UV pour le contrôle de la pureté*

La qualité pharmaceutique de la substance active, l'imiquimod (composé de formule (VII), tel que décrit à la Figure 9), est contrôlée selon les méthodes générales de la Pharmacopée Européenne et selon une méthode CLHP / UV spécifique et indicatrice de stabilité (Hussain S, Shaikh T, and Farooqui M, Development and Validation of Liquid Chromatography Method for the Determination and Quantification of Impurities in Imiquimod. Journal of Pharmaceutical Research International. 13, 1 (Sep. 2016), 1-9).

*Impuretés de synthèse (telles que décrites à la Figure 10)*

Les impuretés recherchées sont principalement :

- L'impureté C ou 4-chloro-1-isobutyl-1H-imidazo[4,5-c]quinoléine ;
- Le catalyseur de synthèse : le cuivre ;
- Des substances apparentées : l'impureté E, intermédiaire de synthèse (N-isobutylquinoléine-3,4-diamine) et impureté A, composé de désamination (1-isobutyl-1H-imidazo[4,5-c]quinoléine).

*Produits de dégradation par dégradation thermique, oxydation ou photooxydation*

Ces impuretés sont les suivantes et sont décrites à la figure 11 :

- L'impureté A (produit de désamination), 1-isobutyl-1H-imidazo[4,5-c]quinoléine ;
- L'impureté B (N-oxyde et désamination), 1-isobutyl-1H-imidazo[4,5-c]quinoléine 5-oxyde ;
- L'impureté N-oxyde, 1-isobutyl-1H-imidazo[4,5-c]quinole-4-amino-5-oxyde.

*Rendement*

Typiquement, le rendement final de la synthèse correspond au rapport (M1) de la masse d'imiquimod (composé de formule (VII)) obtenu après recristallisation, sur la masse du composé de formule (VI) tel que représenté à la Figure 9, (M2), multiplié par le rapport des masses molaires, multiplié par cent, soit la formule ci-après décrite.

[Math.1]

$$rendement\ \% = \frac{M_1}{M_2} \times \frac{MM_2}{MM_1} \times 100$$

avec M1 : masse de l'imiquimod, M2 : masse du composé de formule (VI), MM1 : masse molaire de l'imiquimod et MM2 : masse molaire du composé de formule (VI).

**Exemple 1 : procédé de synthèse de l'imiquimod**

*Etape 1 : nitration de la quinoléine-2,4-diol (obtention du 3-nitroquinolin-2,4-diol, composé de formule (II))*

10,2 g de quinoléine-2,4-diol (0,063 mole) (composé de formule (I) de la Figure 9) sont nitrés avec 40 ml d'acide nitrique concentré (minimum 65 %) (0,870 mole) par chauffage à 75°C sans solvant pendant 15 minutes.

Après refroidissement, la solution est hydrolysée avec 400 ml d'eau glacée et laissée sous agitation pendant 15

minutes. Après précipitation, le produit est isolé par filtration et lavage à l'eau glacé (2 x 250 ml) (rendement 95 %).

Le 3-nitroquinolin-2,4-diol (composé de formule (II) de la Figure 9) obtenu est identifié par les méthodes précédemment décrites.

*Etape 2 : chloration en position 2,4 de la 3-nitroquinolein-2,4-diol (obtention du composé III de la Figure 9)*

42 ml de trichlorure de phosphore (0,45 mole) sont ajoutés au composé de formule (II) tel que décrit à la Figure 9. Le mélange réactionnel est chauffé à 90°C pendant 2h.

Après refroidissement, la solution est hydrolysée avec 300 ml de glace et laissée sous agitation. Après précipitation, le produit est isolé par filtration. Le produit filtré est repris dans le dichlorométhane et filtré sur silice. Le filtrat est évaporé à sec (rendement 80 %).

Le composé de formule (III) ainsi obtenu est identifié par les méthodes précédemment décrites.

*Etape 3 : substitution nucléophile du composé de formule (III) avec l'isobutylamine en position 4 (obtention du composé de formule (IV) de la Figure 9)*

10 g de composé de formule (III) (tel que décrit à la Figure 9) (0,041 mole) est solubilisé dans 50 ml de dichlorométhane. La triéthylamine (8,6 ml, 0,062 mole) puis l'isobutylamine (4,8 ml, 0,048 mole) sont ajoutées. Le mélange est chauffé à 45°C pendant 30 minutes. Le solvant est évaporé et le résidu est précipité avec 100 ml d'eau puis filtré. Le produit est lavé avec 130 ml de cyclohexane et de nouveau filtré (rendement 95 %).

Le composé de formule (IV) ainsi obtenu est identifié par les méthodes précédemment décrites.

*Etape 4 : réduction du dérivé nitré par hydrogénation catalytique (obtention du composé de formule (V) de la Figure 9)*

Le composé de formule (IV) (6,5 g, 0,023 mole) dans 200 ml acétate d'éthyle est additionné de sulfate de sodium (0,035 mole). Pd/C (371 mg, 0,00349 mole) est additionné au mélange qui est hydrogéné pendant 5h. Le mélange est filtré sur celite et le solvant est évaporé (rendement 95 %).

Le composé de formule (V) ainsi obtenu est identifié par les méthodes précédemment décrites.

*Etape 5 : cyclisation avec le triéthylorthoformiate et formation du noyau imidazole (obtention du composé de formule (VI) de la Figure 9)*

Un mélange de triéthylorthoformiate (4,53 ml, 0,027 mole) et du composé de formule (V) précédemment obtenu (4,54 g, 0,018 mole) dans 50 ml de toluène est chauffé à 110°C pendant 2h. Après refroidissement, le solvant est évaporé puis le résidu est précipité avec 40 ml d'isopropanol. Après filtration, le produit est lavé avec 40 ml d'éther (rendement 65 %).

Le composé de formule (VI) ainsi obtenu est identifié par les méthodes précédemment décrites.

*Etape 6 : substitution nucléophile du composé de formule (VI) par l'ammoniaque en position 2 (obtention de l'imiquimod (composé de formule (VII) de la Figure 9))*

Un mélange du composé de formule (VI) précédemment obtenu (10,06 g, 0,039 mole), de CuCl(I) (396,6 mg, 0,004 mole) dans 130 ml d'une solution d'ammoniaque dans le méthanol 7N (≈ 12 %) est chauffé à 145°C dans une cuve sous pression (10 - 15 bars) pendant 7h. Après refroidissement, le mélange réactionnel est filtré puis lavé successivement avec du méthanol, de l'eau et du méthanol. Le produit obtenu est finalement recristallisé dans le DMF (rendement 85 %).

L'imiquimod (composé de formule (VII) de la Figure 9) obtenu est identifié par les méthodes précédemment décrites.

## Exemple 2 : Analyse et contrôle de la pureté de l'imiquimod obtenu selon le procédé de synthèse selon l'invention et son rendement

La qualité pharmaceutique de l'imiquimod est contrôlée par la méthode CLHP/UV précédemment décrite.

Les impuretés les plus caractéristiques et les plus largement représentées dans ce type de préparation de l'imiquimod sont l'impureté A (1-isobutyl-1H-imidazo[4,5-c]quinoléine), l'impureté C (4-chloro-1-isobutyl-1H-imidazo[4,5-c]quinoléine) et l'impureté E (N-isobutylquinoléine-3,4-diamine) décrites par Sayyed Hussain et al. (Hussain S, Shaikh T, and Farooqui M, Development and Validation of Liquid Chromatography Method for the Determination and Quantification of Impurities in Imiquimod. Journal of Pharmaceutical Research International. 13, 1 (Sep. 2016), 1-9).

Il a été mesuré, par la méthode précédemment citée, que l'imiquimod obtenu selon le procédé de l'invention

présente une pureté de 99,8%.

Les autres impuretés identifiées comme substances apparentées ou produits d'oxydation n'ont pas été retrouvées dans le produit final.

De tels niveaux d'impuretés satisfont totalement aux exigences requises par la Pharmacopée Européenne et les recommandations des guidelines ICH Q3A, Q3C et Q3D.

Rendement

Le rendement final de la synthèse correspond au rapport (M1) de la masse d'imiquimod obtenu après recristallisation sur la masse du composé de formule (VI) tel que représenté à la Figure 9 (M2), multiplié par le rapport des masses molaires, multiplié par cent. La formule est ci-après décrite.

[Math.2]

$$rendement\ \% = \frac{M_1}{M_2} \times \frac{MM_2}{MM_1} \times 100$$

avec M1 : masse de l'imiquimod, M2: masse du composé de formule (VI), MM1 : masse molaire de l'imiquimod et MM2 : masse molaire du composé VI.

Le rendement est de 85%.

**Exemple** 3 : **Analyse comparative des profils d'impuretés de l'imiquimod obtenu selon les procédés de l'état de la technique - sans utilisation de catalyseur métallique - et selon le procédé objet de l'invention - avec catalyseur métallique**

La pureté de l'imiquimod obtenu par le procédé selon l'invention a été comparée à la pureté de l'imiquimod obtenu par les procédés de l'état de la technique, à savoir les procédés décrits dans les documents US 4,689,338, J. Med. Chem. 2005, 48, 3481-3491 et ES 2 538 880.

Matériels et Méthodes

Principe :

Lot IMI01 : Imiquimod de qualité pharmaceutique obtenu selon le procédé objet de l'invention obtenu à partir du composé chloré (composé VI) en présence de chlorure de cuivre.

Lot IMI02 : Imiquimod préparé selon la méthode des brevets US 4,689,338 (GERSTER), ES 2 538 880 (VINAS) et l'article J. Med. Chem 2005, 48, 3481-3491 à partir du composé chloré (composé VI) sans catalyseur métallique.

L'analyse de pureté de l'imiquimod préparé avec et sans utilisation de catalyseur a été réalisée par CLHP/UV en accord avec les exigences ICH Q3A pour les impuretés organiques des substances actives et qui reprend le profil d'impuretés décrits par Sayyed et al.

Préparation des solutions :

**Diluant**

Mélange équimoléculaire de solution aqueuse d'acide phosphorique à 1 % et d'acétonitrile (50V/50V).

**Eluant**

Phase mobile A : solution aqueuse d'acide phosphorique à 1 %
Phase mobile B : acétonitrile R.

**Solution témoin (T1)**

Prendre une prise d'essai d'environ 100,0 mg d'Imiquimod de référence exactement pesée et diluer à 100 ml avec le diluant.

**Solution témoin (T2)(0,1%)**

Diluer la solution T1 obtenue au 1/1000 dans le diluant

**Solution d'essai (E)**

Prendre une prise d'essai d'environ 100,0 mg d'Imiquimod de référence exactement pesée et diluer à 100 ml avec le diluant.

Technique

Un exemple de conditions opératoires pratiquées avec le matériel du laboratoire est décrit ci-après. Elles peuvent varier légèrement selon le matériel utilisé. Dans une chaîne chromatographique convenablement équipée et réglée, injecter exactement 10 µL de chacune des solutions témoin et essai.

Calcul soient :

$A_1$ : la valeur de l'aire du pic d'impureté obtenu pour la solution témoin ($T_2$)
$A_2$ : la valeur de l'aire du pic d'impureté obtenu pour la solution essai (E),
$P_1$ : la prise d'essai de l'imiquimod de référence en mg.

La teneur en % d'impureté de l'imiquimod sera donnée par l'expression

$$t_1 = (A_2 / A_1) \text{ X } (P_1 / 1000) \text{ en } \%$$

Exemple de conditions opératoires par chromatographie liquide haute performance :

| | |
|---|---|
| **Produit :** | Imiquimod |
| **Analyse :** | Synthèse de l'imiquimod |
| **Appareillage :** | Système LACHROM ELITE<br>Pompe L2130<br>Four L2300<br>Injecteur L2200<br>Détecteur L2400<br>Logiciel LACHROM ELITE |
| **Colonne :** | ZORBAX SB-C18 (250 x 3 mm) - 5 µm<br>Réf Agilent 880975-302 |
| **Eluant :** | |

| Intervalle (min) | Phase mobile A (pour cent V/V) | Phase mobile B (pour cent V/V) |
|---|---|---|
| 0-25 | 90 | 10 |
| 25-30 | 30 | 70 |
| 30-40 | 90 | 10 |

| | |
|---|---|
| **Température colonne :** | 30°C |
| **Débit :** | 1,0 ml.min$^{-1}$ |
| **Détection** : | 260 nm |
| **Volume injecté** : | 10 µL |

(suite)

| | |
|---|---|
| **Temps de rétention** : | Imiquimod : $T_R \cong 8,1$ minutes |
| | Dérivé chloré (impureté C) : $T_R \cong 10,3$ minutes (RR = 1,2) |

Résultats :

Le chromatogramme de l'imiquimod obtenu selon le procédé selon l'invention (lot IMI01) est représenté à la Figure 12.

Le chromatogramme de l'imiquimod obtenu selon les procédés de l'état de la technique (lot IMI02) est représenté à la Figure 13.

Le chromatogramme du blanc (solution de dilution) est représenté à la Figure 14 et le chromatogramme du témoin de référence (imiquimod à 0,1%, de TCI Chemicals) est représenté à la Figure 15.

Les teneurs en impuretés de l'imiquimod obtenu selon le procédé selon l'invention (lot IMI01) et selon les procédés de l'état de la technique (lot IMI02), sont représentés dans le tableau ci-après :

| Essais | Spécifications (ICH Q3A) | Analyse Lot IMI01 | Analyse Lot IMI02 |
|---|---|---|---|
| **Recherche des impuretés par CLHP** | | | |
| ▪ Impureté C (dérivé chloré) | ≤ 0,15 % | 0 ,1 % | 1,9 % |
| ▪ Impureté TR = 5,2 | ≤ 0,15 % | < 0,05 % | 0,1 % |
| ▪ Impureté TR = 9,4 | ≤ 0,15 % | 0,1 % | 0,4 % |
| ▪ Impureté TR = 12,5 | ≤ 0,15 % | < 0,05 % | 0,1 % |
| ▪ Impureté TR = 13,5 | ≤ 0,15 % | < 0,05 % | 0,2 % |
| ▪ Impureté TR = 16,4 | ≤ 0,15 % | < 0,05 % | 0,2 % |
| Total des impuretés | ≤ 0,5 % | 0,2 % | 2,9 % |
| **Teneur en substance active** | | | |
| (HPLC) | ≥ 98.0 % | 99.8 % | 97.1 % |
| **CONCLUSION** | | **CONFORME** | **NON CONFORME** |

Il apparait clairement que le profil chromatographique obtenu pour l'imiquimod préparé selon le procédé objet de la présente demande et comprenant donc l'utilisation d'un catalyseur à base de cuivre (CuCl) (lot IMI01) montre aucune impureté organique supérieure à 0,1% et une teneur en substance active supérieure à 99,8 % (le seuil maximal ICH Q3A acceptable pour le taux d'impureté non qualifiée dans une substance active est de 0,15 %).

En revanche, le profil chromatographique obtenu pour l'imiquimod préparé selon les procédés décrits dans l'état de la technique, sans utilisation de catalyseur à base de cuivre (lot IMI02) montre plusieurs impuretés organiques supérieures à 0,2 % et une teneur en substance active inférieure ou égale à 97,1 %. En particulier on observe une teneur importante en dérivé chloré (impureté C), produit de départ de la dernière étape de synthèse (1,9 %) non compatible à l'utilisation d'un produit de qualité pharmaceutique.

Le procédé selon l'invention permet avantageusement de générer une substance active, l'imiquimod, de haute pureté, utilisable comme molécule de qualité pharmaceutique répondant aux exigences de la Pharmacopée Européenne et des recommandations guidelines ICH Q3A, Q3C et Q3D.

## Revendications

1. Procédé de synthèse d'un composé de formule (VII)

[Chem 1]

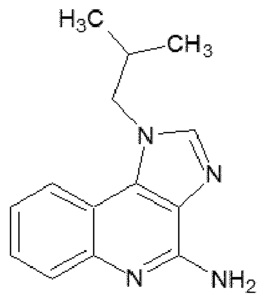

(VII)

**caractérisé en ce qu'**il comprend une étape de substitution nucléophile aromatique par aminolyse d'un composé de formule (VI)

[Chem 2]

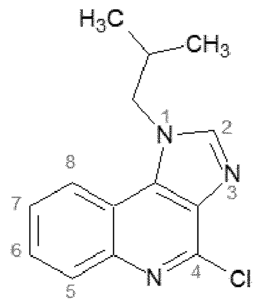

(VI)

,

ladite étape de substitution nucléophile aromatique par aminolyse étant réalisée en présence d'au moins un catalyseur métallique choisi parmi les sels de cuivre, les oxydes cuivriques, les oxydes cuivreux et leurs mélanges.

**2.** Procédé selon la revendication 1, **caractérisée en ce qu'**il comprend en outre, une étape de substitution nucléophile d'un composé de formule (III)

[Chem 3]

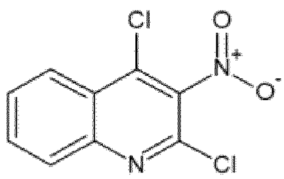

composé (III)

pour l'obtention d'un composé de formule (IV),

[Chem 4]

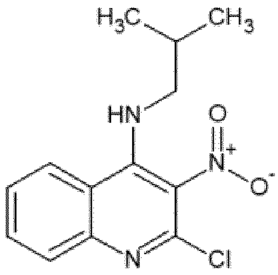

composé (IV)

,

ladite étape de substitution nucléophile étant réalisée en présence d'isobutylamine, d'au moins un catalyseur tel que la triéthylamine et d'au moins un solvant polaire aprotique.

3. Procédé selon les revendications 1 ou 2, **caractérisé en ce qu'**il comprend un première étape, ladite première étape étant une étape de nitration de la quinoline-2,4-diol, de formule (I)

[Chem 5]

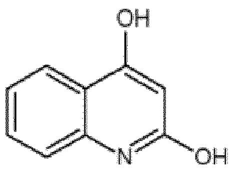

(I)

*pour l'obtention* d'un composé de formule (II)

[Chem 6]

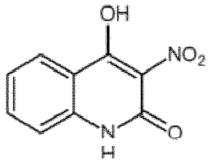

(II)

4. Procédé selon la revendication 3, **caractérisé en ce qu'**il comprend une seconde étape, ladite seconde étape étant une étape de chloration en position 2 et 4 du composé de formule (II) pour l'obtention d'un composé de formule (III).

5. Procédé selon la revendication 4, **caractérisé en ce qu'**il comprend une troisième étape, ladite troisième étape étant une étape de substitution nucléophile selon la revendication 2.

6. Procédé selon la revendication 4, **caractérisé en ce qu'**il comprend une quatrième étape, ladite quatrième étape étant une étape de réduction par hydrogénation catalytique du composé de formule (IV) pour l'obtention d'un composé de formule (V)

[Chem 7]

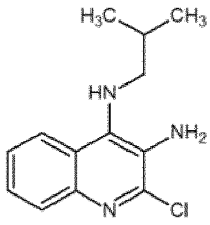

composé (V)

.

7. Procédé selon la revendication 6, **caractérisé en ce qu'**il comprend une cinquième étape, ladite cinquième étape étant une étape de cyclisation à partir du composé de formule (V) pour l'obtention d'un composé de formule (VI)

[Chem 8]

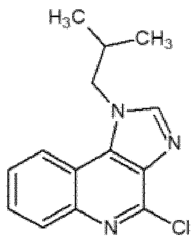

composé (VI)

,

ladite cyclisation étant réalisée en présence de triéthylorthoformiate.

8. Procédé selon la revendication 7, **caractérisé en ce qu'**il comprend une sixième étape, ladite sixième étape étant l'étape de substitution nucléophile aromatique par aminolyse selon la revendication 1.

## Patentansprüche

1. Verfahren zur Herstellung einer Verbindung der Formel (VII)

[Chem 1]

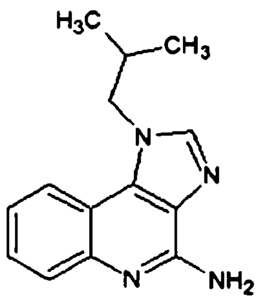

(VII)

**dadurch gekennzeichnet, dass** es einen Schritt der nukleophilen aromatischen Substitution durch Aminolyse einer Verbindung der Formel (VI) umfasst.

[Chem 2]

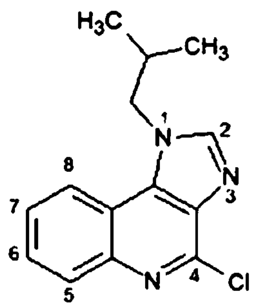

(VI)

wobei der Schritt der nukleophilen aromatischen Substitution durch Aminolyse in Gegenwart mindestens eines metallischen Katalysators durchgeführt wird, der ausgewählt ist aus Kupfersalzen, Kupfer(II)-oxiden, Kupfer(I)-oxiden und Mischungen davon.

2. Verfahren nach Anspruch1, **dadurch gekennzeichnet, dass** es weiterhin einen Schritt der nukleophilen Substitution einer Verbindung der Formel (III) umfasst

[Chem 3]

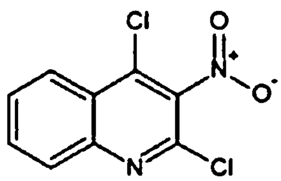

Verbindung (III)

um eine Verbindung der Formel (IV) zu erhalten,

[Chem 4]

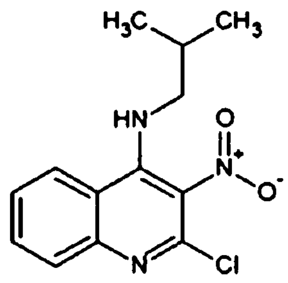

Verbindung (IV)

wobei der Schritt der nukleophilen Substitution in Gegenwart von Isobutylamin, mindestens einem Katalysator wie Triethylamin und mindestens einem polaren aprotischen Lösungsmittels durchgeführt wird,

3. Verfahren nach Anspruch 1 oder 2, **dadurch gekennzeichnet, dass** es einen ersten Schritt umfasst, wobei der erste Schritt ein Schritt der Nitrierung von Chinolin-2,4-diol der Formel (I) ist

[Chem 5]

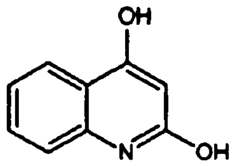

(I)

um eine Verbindung der Formel (II) zu erhalten

[Chem 6]

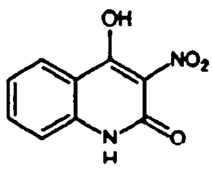

(II)

4. Verfahren nach Anspruch 3, **dadurch gekennzeichnet, dass** es einen zweiten Schritt umfasst, wobei der zweite Schritt ein Schritt der Chlorierung der Verbindung der Formel (II) an Position 2 und 4 ist, um eine Verbindung der Formel (III) zu erhalten.

5. Verfahren nach Anspruch 4, **dadurch gekennzeichnet, dass** es einen dritten Schritt umfasst, wobei der dritte Schritt ein Schritt der nukleophilen Substitution nach Anspruch 2 ist.

6. Verfahren nach Anspruch 4, **dadurch gekennzeichnet, dass** es einen vierten Schritt umfasst, wobei der vierte Schritt ein Schritt der Reduktion durch katalytische Hydrierung der Verbindung der Formel (IV) ist, um eine Verbindung der Formel (V) zu erhalten

[Chem 7]

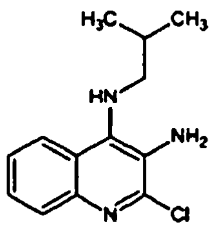

Verbindung (V).

7. Verfahren nach Anspruch 6, **dadurch gekennzeichnet, dass** es einen fünften Schritt umfasst, wobei der fünfte Schritt ein Schritt der Zyklisierung ausgehend von einer Verbindung der Formel (V) ist, um eine Verbindung der Formel (VI) zu erhalten

[Chem 8]

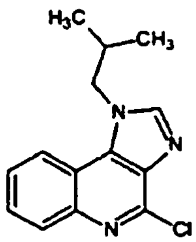

Verbindung (VI)

wobei die Zyklisierung in Gegenwart von Triethylorthoformiat durchgeführt wird.

8. Verfahren nach Anspruch 7, **dadurch gekennzeichnet, dass** es einen sechsten Schritt umfasst, wobei der sechste Schritt der Schritt der nukleophilen aromatischen Substitution durch Aminolyse nach Anspruch 1 ist.

## Claims

1. A process for synthesising a compound of the formula (VII) [Chem 1]

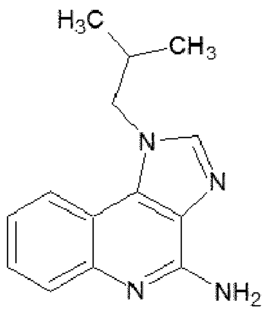

(VII)

**characterised in that** it comprises an aromatic nucleophilic substitution step by aminolysis of a compound of the formula (VI)

[Chem 2]

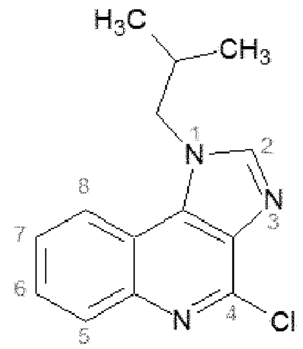

(VI)

,

said aromatic nucleophilic substitution step by aminolysis being carried out in the presence of at least one metal catalyst selected from copper salts, cupric oxides, cuprous oxides, and mixtures thereof.

**2.** The process according to claim 1, **characterised in that** it further comprises a nucleophilic substitution step for a compound of the formula (III)

[Chem 3]

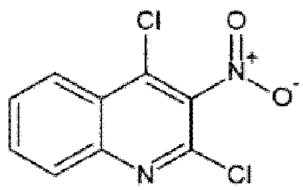

composé (III)

for obtaining a compound of the formula (IV), [Chem 4]

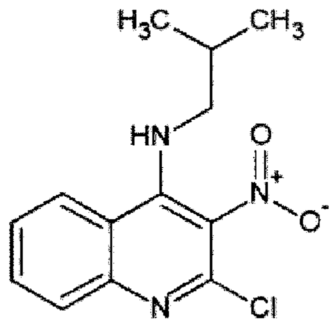

composé (IV)

,

said nucleophilic substitution step being carried out in the presence of isobutylamine, at least one catalyst such as triethylamine and at least one aprotic polar solvent.

**3.** The process according to claims 1 or 2, **characterised in that** it comprises a first step, said first step being a step of nitrating quinoline-2.4-diol, of the formula (I) [Chem 5]

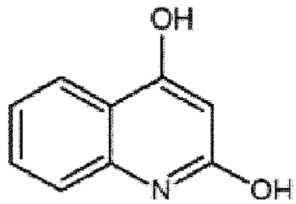

(I)

for obtaining a compound of the formula (II) [Chem 6]

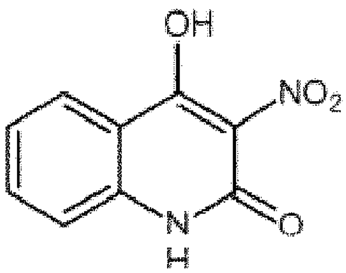

(II)

**4.** The process according to claim 3, **characterised in that** it comprises a second step, said second step being a step of chlorinating the compound of the formula (II) at positions 2 and 4 for obtaining a compound of the formula (III).

**5.** The process according to claim 4, **characterised in that** it comprises a third step, said third step being a nucleophilic substitution step according to claim 2.

**6.** The process according to claim 4, **characterised in that** it comprises a fourth step, said fourth step being a step of reducing the compound of the formula (IV) by catalytic hydrogenation for obtaining a compound of the formula (V) [Chem 7]

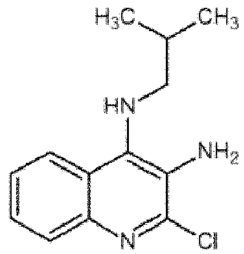

composé (V)

7. The process according to claim 6, **characterised in that** it comprises a fifth step, said fifth step being a cyclisation step from the compound of the formula (V) for obtaining a compound of the formula (VI) [Chem 8]

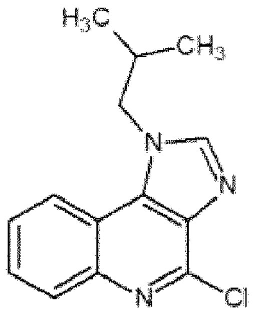

composé (VI)

,

said cyclisation being carried out in the presence of triethyl orthoformate.

8. The process according to claim 7, **characterised in that** it comprises a sixth step, said sixth step being the aromatic nucleophilic substitution step by aminolysis according to claim 1.

[Fig. 1]

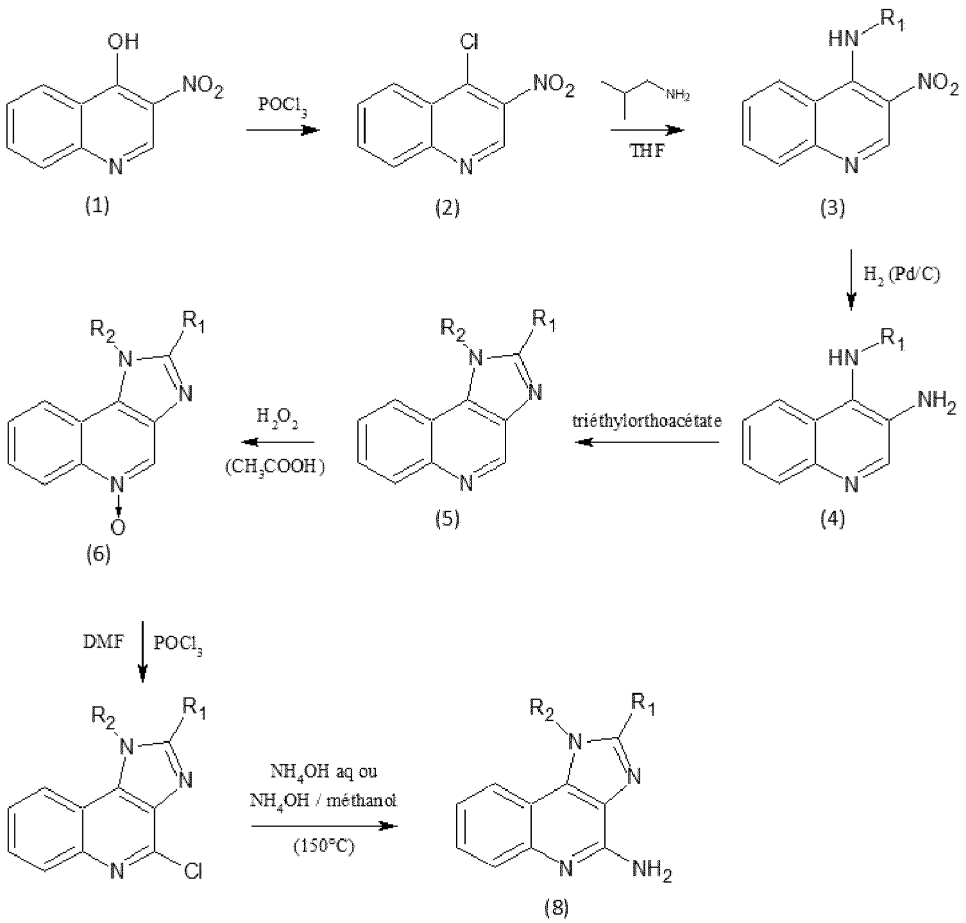


(1) (2) (3) (4) (5) (6) (7) (8)

R1 = H , R2 = isobutyl
Imiquimod

[Fig. 2]

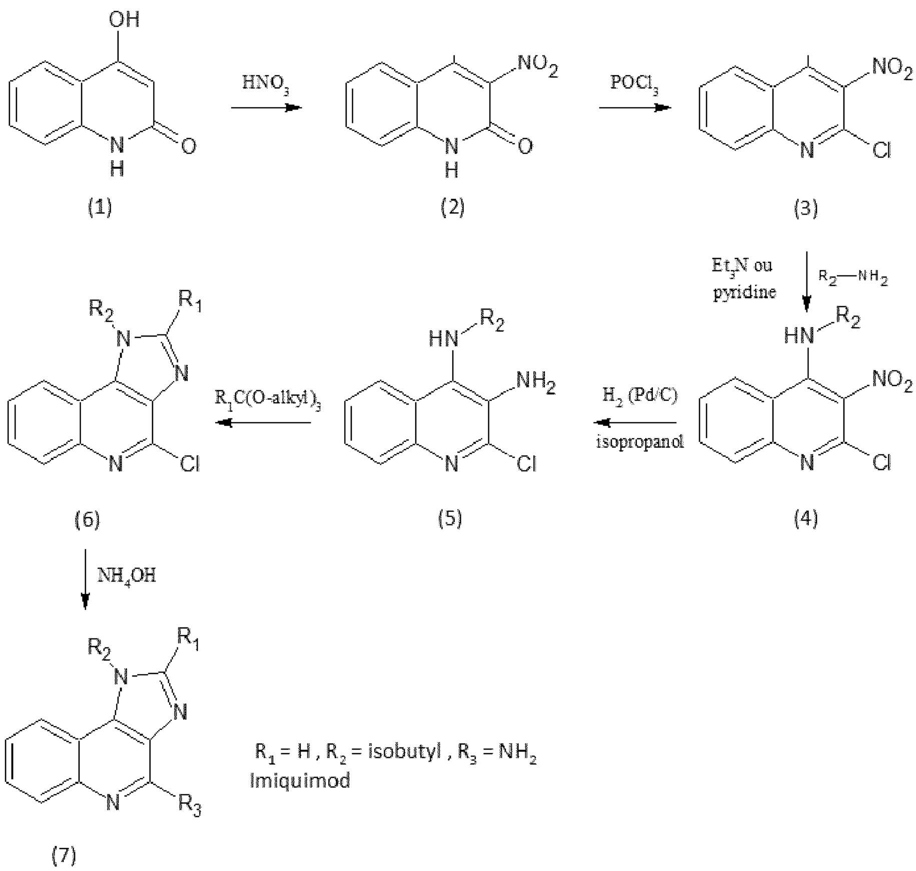


(1) (2) (3)

(6) (5) (4)

(7)

$R_1$ = H , $R_2$ = isobutyl , $R_3$ = $NH_2$
Imiquimod

[Fig. 3]

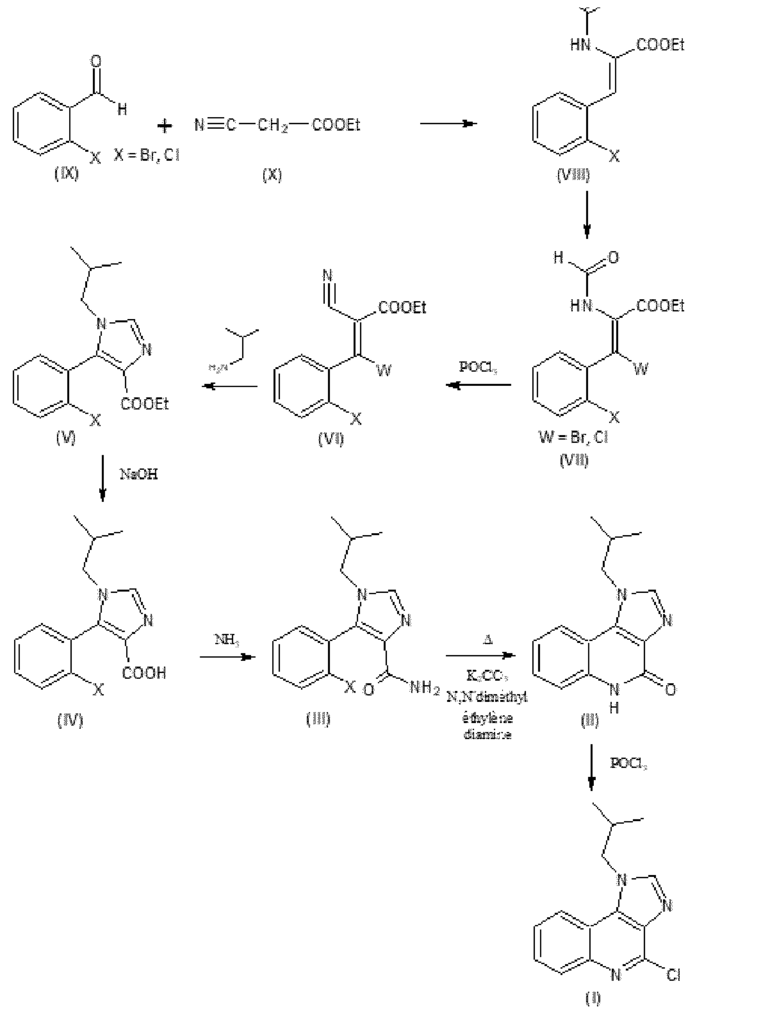


1-isobutyl-4-chloro-1H-imidazo[4,5-c]quinoléine

[Fig. 4]

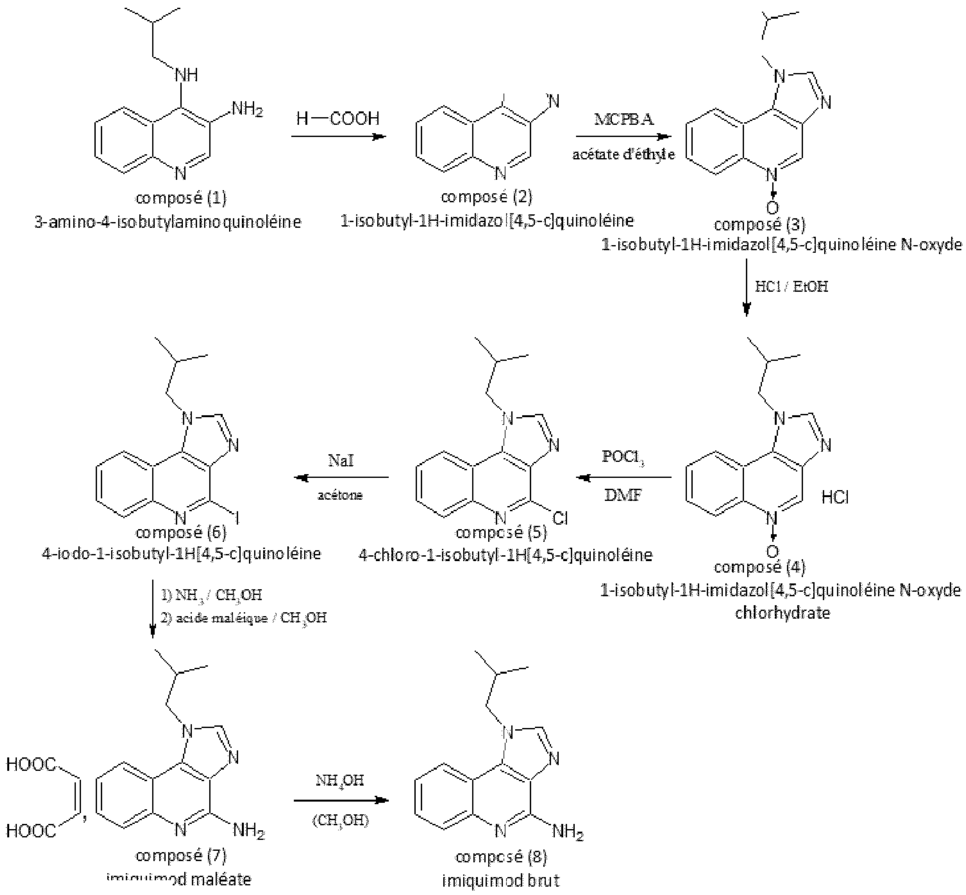


composé (1)
3-amino-4-isobutylaminoquinoléine

composé (2)
1-isobutyl-1H-imidazol[4,5-c]quinoléine

composé (3)
1-isobutyl-1H-imidazol[4,5-c]quinoléine N-oxyde

composé (4)
1-isobutyl-1H-imidazol[4,5-c]quinoléine N-oxyde chlorhydrate

composé (5)
4-chloro-1-isobutyl-1H[4,5-c]quinoléine

composé (6)
4-iodo-1-isobutyl-1H[4,5-c]quinoléine

composé (7)
imiquimod maléate

composé (8)
imiquimod brut

[Fig. 5]

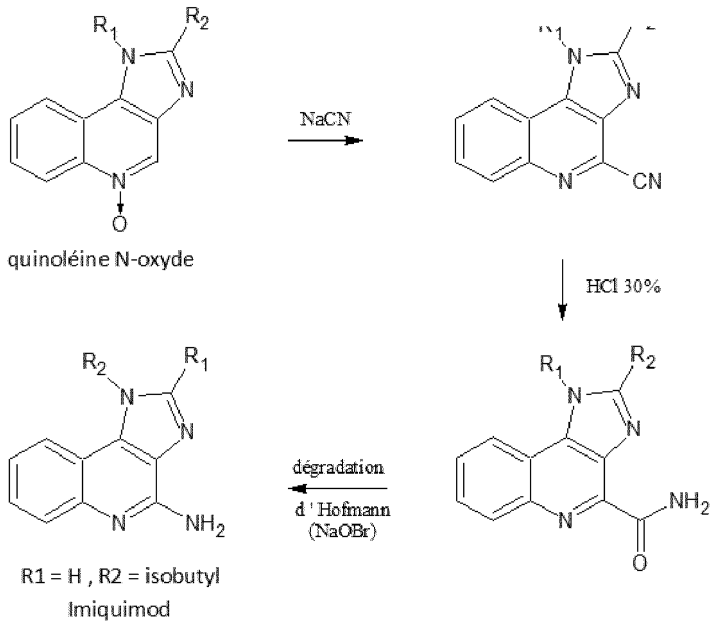
NaCN
HCl 30%
dégradation
d ' Hofmann
(NaOBr)
quinoléine N-oxyde
R1 = H , R2 = isobutyl
Imiquimod

[Fig. 6]

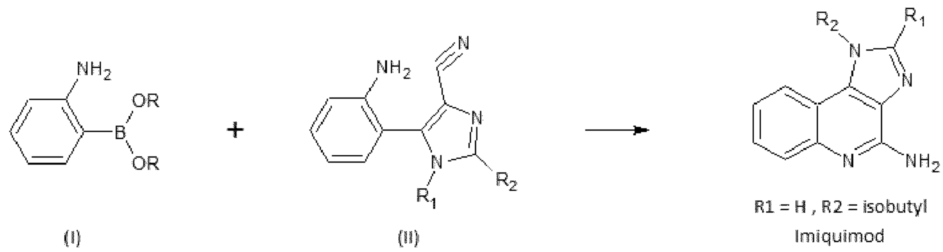
(I)
(II)
R1 = H , R2 = isobutyl
Imiquimod

[Fig. 7]

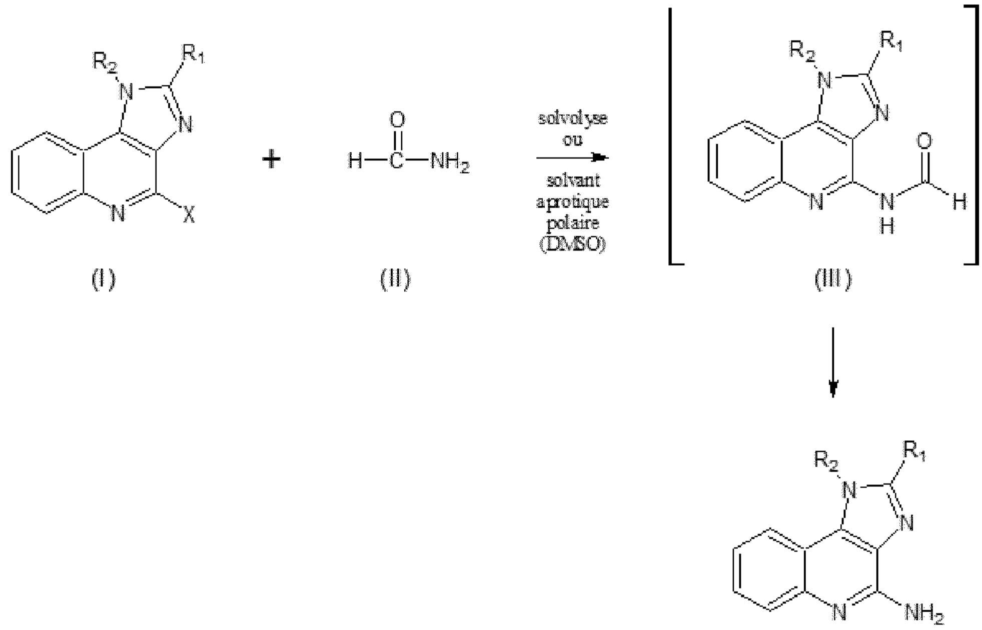


(I) (II) (III)

(IV)

$R_1$ = H , $R_2$ = isobutyle

Imiquimod

[Fig. 8]

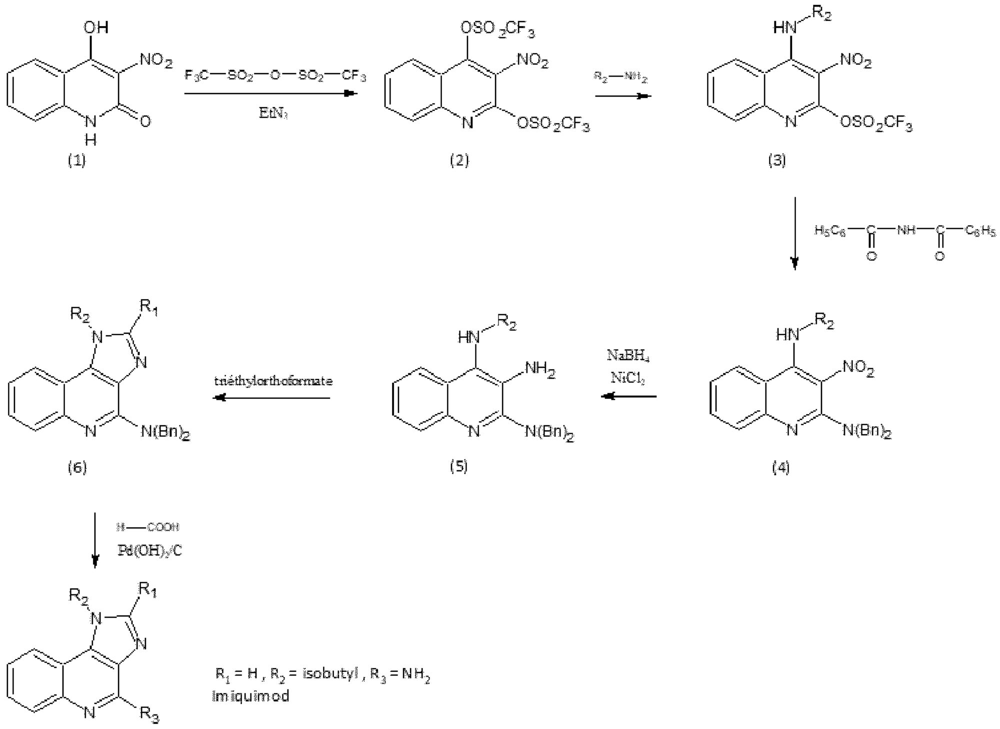


(1) (2) (3) (4) (5) (6) (7)

$R_1$ = H , $R_2$ = isobutyl , $R_3$ = $NH_2$
Imiquimod

[Fig. 9]

1- Etape 1 : nitration de la quinoline

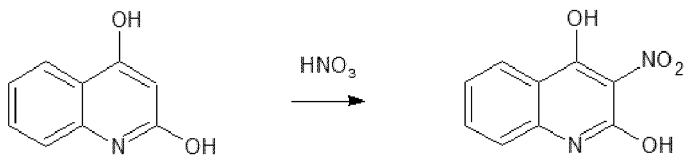


composé (I)
quinoline-2,4-diol
CAS : 86-95-3

composé (II)

2- Etape 2 : chloration du composé (II) en position 2 et 4

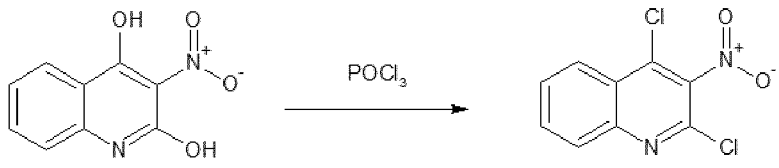


composé (II)

composé (III)

3- Etape 3 : substitution nucléophile du composé (III) avec l'isobutylamine en position 4

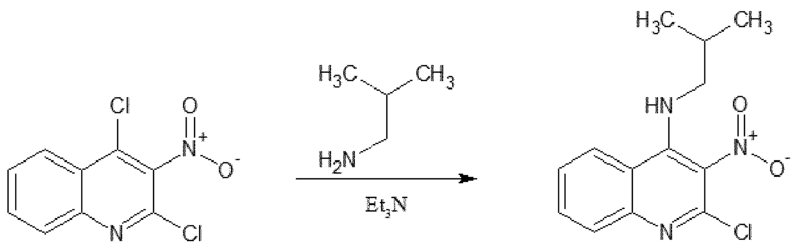


composé (III)

composé (IV)

4- Etape 4 : réduction du dérivé nitré par hydrogénation catalytique

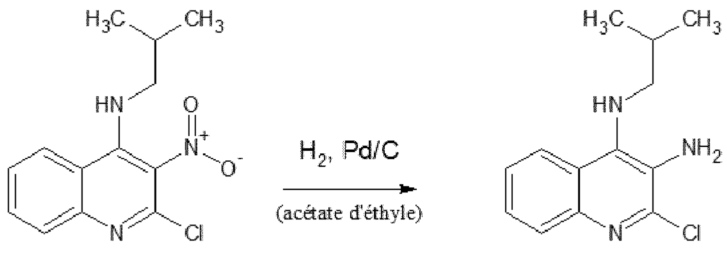


composé (IV) composé (V)

5- Etape 5 : cyclisation avec le triétylorthoformiate et formation du noyau imidazole

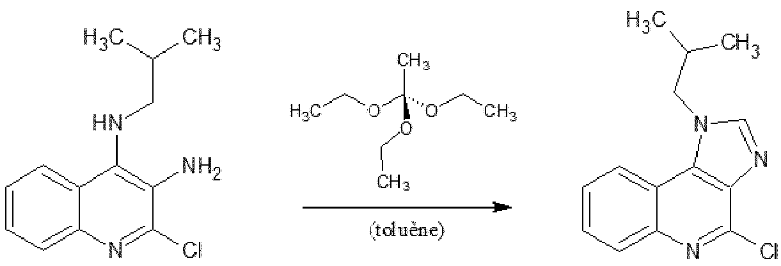


composé (V) composé (VI)

6- Etape 6 : substitution nucléophile du composé (VI) par l'ammoniaque en position 2

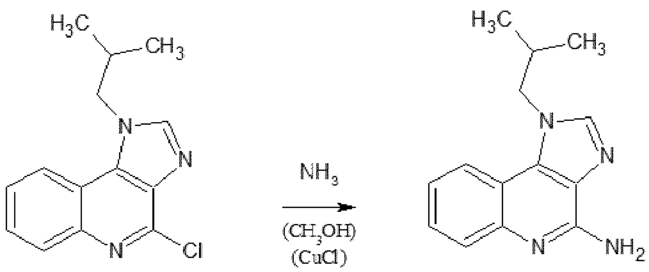


composé (VI) composé (VII)
Imiquimod

[Fig. 10]

| NOM | STRUCTURE | NUMERO CAS | FORMULE BRUTE | MASSE MOLAIRE |
|---|---|---|---|---|
| Impureté A | 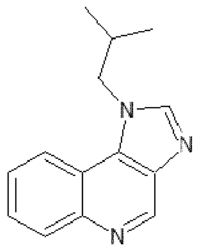 | 99010-24-9 | $C_{14}H_{15}N_3$ | 225,29 |
| Impureté C | 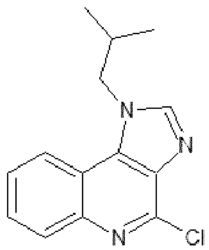 | 99010-64-7 | $C_{14}H_{14}ClN_3$ | 259,73 |
| Impureté E | 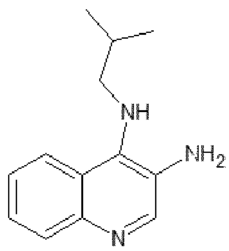 | 99010-09-0 | $C_{17}H_{17}N_3$ | 215,29 |

[Fig. 11]

| NOM | STRUCTURE | NUMERO CAS | FORMULE BRUTE | MASSE MOLAIRE |
|---|---|---|---|---|
| Impureté A | 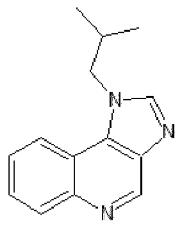 | 99010-24-9 | $C_{14}H_{15}N_3$ | 225,29 |
| Impureté B | 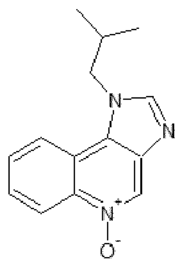 | 99010-63-6 | $C_{14}H_{15}N_3O$ | 241,29 |
| Impureté N-Oxyde | 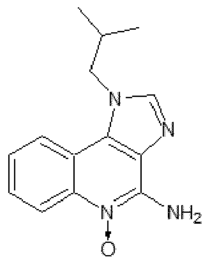 | | $C_{14}H_{16}N_4O$ | 256,30 |

[Fig.12]

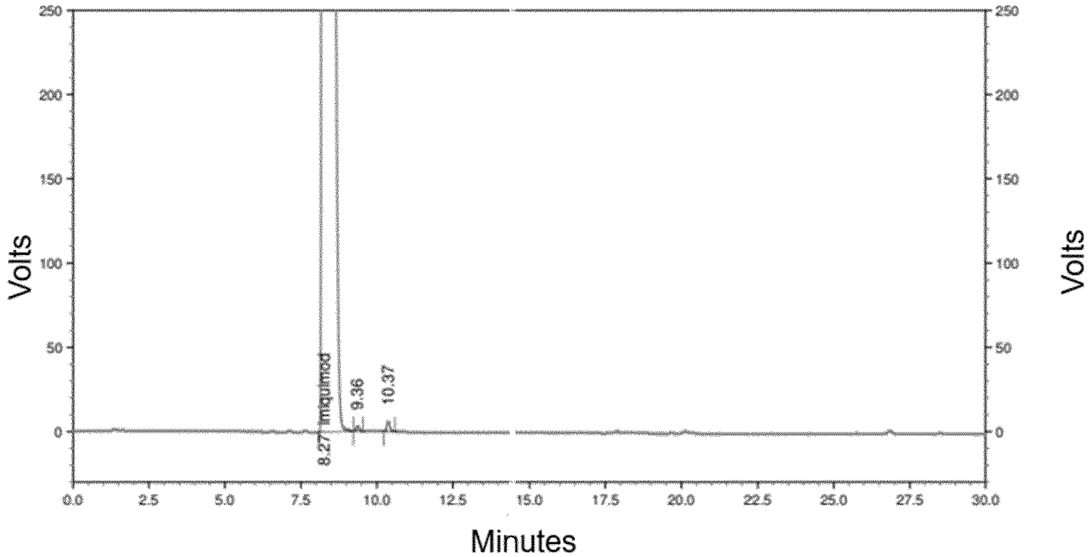


| Résultats UV N° | *Temps (min)* | *Nom* | *Surface* | Code inter. | Pourcentage de surface |
|---|---|---|---|---|---|
| 1 | 8.27 | imiquimod | 116844407 | vv | 99.8 |
| 2 | 9.36 | | 84699 | vv | 0.1 |
| 3 | 10.37 | | 168886 | vv | 0.1 |

[Fig.13]

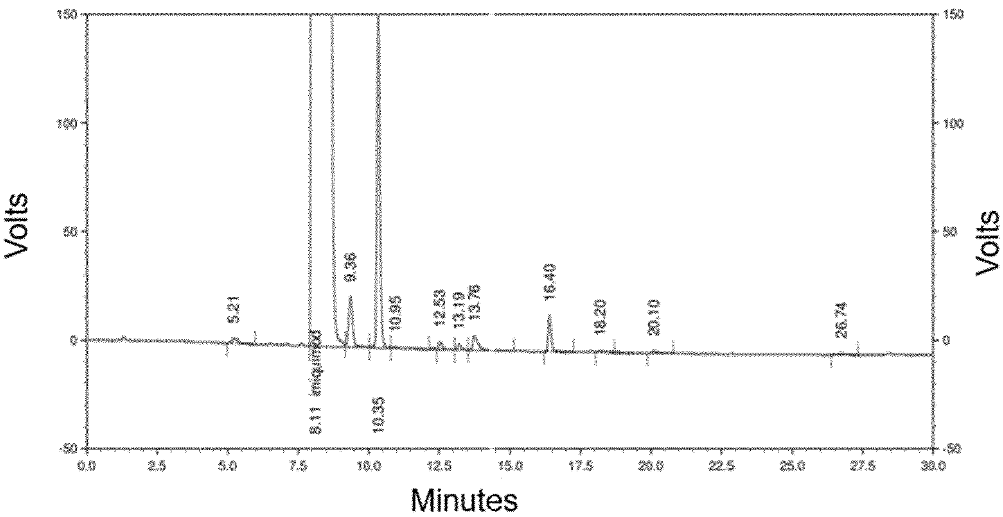


| Résultats UV N° | *Temps (min)* | *Nom* | *Surface* | Code inter. | Pourcentage de surface |
|---|---|---|---|---|---|
| 1 | 5.21 | | 126706 | BB | 0.1 |
| 2 | 8.11 | imiquimod | 230025990 | BV | 97.1 |
| 3 | 9.36 | | 932307 | VV | 0.4 |
| 4 | 10.35 | | 4454145 | VV | 1.9 |
| 5 | 10.95 | | 57559 | VV | 0.0 |
| 6 | 12.53 | | 121071 | VB | 0.1 |
| 7 | 13.19 | | 96024 | BV | 0.0 |
| 8 | 13.76 | | 368497 | VB | 0.2 |
| 9 | 16.40 | | 507000 | BB | 0.2 |
| 10 | 18.20 | | 50779 | BV | 0.0 |
| 11 | 20.10 | | 65310 | VB | 0.0 |
| 12 | 26.74 | | 55778 | BB | 0.0 |

[Fig.14]

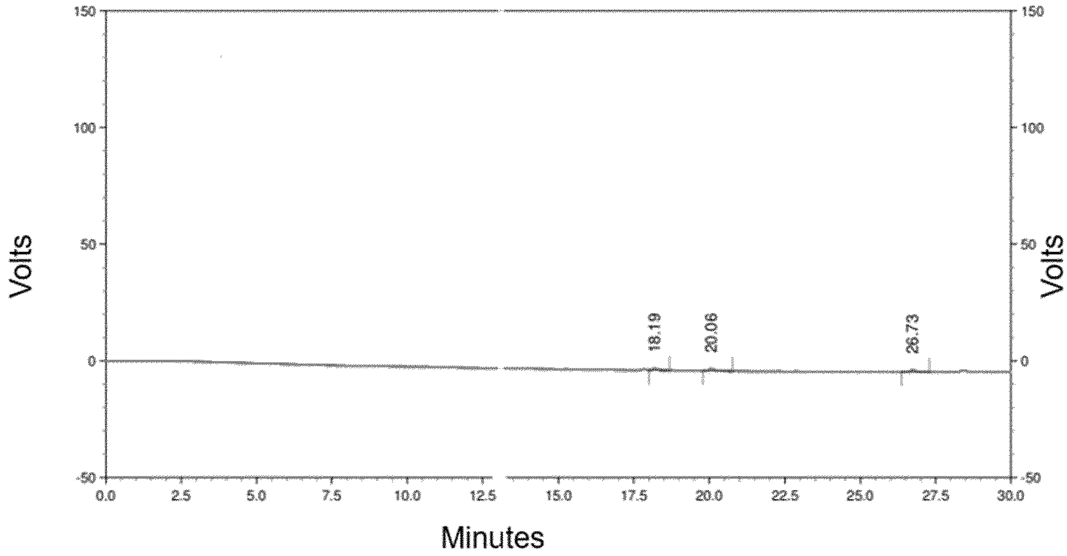


| Résultats UV N° | Temps (min) | Nom | Surface | Code inter. | Pourcentage de surface |
|---|---|---|---|---|---|
| 1 | 18.19 | | 50422 | BB | 30.8 |
| 2 | 20.06 | | 57537 | BB | 35.2 |
| 3 | 26.73 | | 55512 | BB | 34.0 |

[Fig. 15]

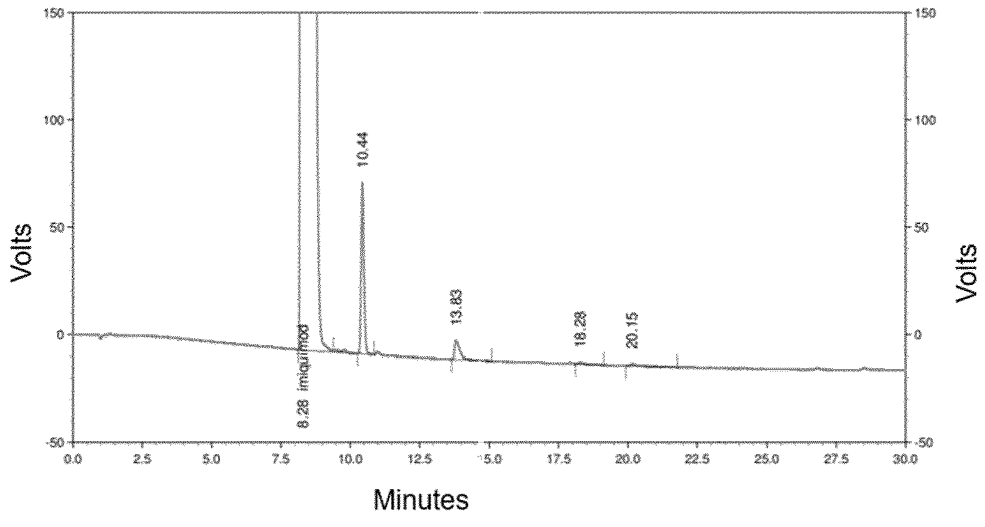


| Résultats UV N° | Temps (min) | Nom | Surface | Code inter. | Pourcentage de surface |
|---|---|---|---|---|---|
| 1 | 8.28 | imiquimod | 160328284 | BV | 98.3 |
| 2 | 10.44 | | 2216570 | VB | 1.4 |
| 3 | 13.83 | | 464286 | BB | 0.3 |
| 4 | 18.28 | | 54640 | BB | 0.0 |
| 5 | 20.15 | | 67504 | BB | 0.0 |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0145340 A
- EP 0425306 A
- US 4689338 A
- US 20080161573 A
- US 7678912 B, Tarur
- US 20090209764 A
- WO 2004011462 A
- EP 2009002 A
- WO 2006100226 A
- WO 9417043 A
- ES 2538880


### Littérature non-brevet citée dans la description


- *J. Med. Chem.*, 2005, vol. 48, 3481-3491
- *CHEMICAL ABSTRACTS*, 86-95-3
- **HUSSAIN S** ; **SHAIKH T** ; **FAROOQUI M**. Development and Validation of Liquid Chromatography Method for the Determination and Quantification of Impurities in Imiquimod. *Journal of Pharmaceutical Research International*, September 2016, vol. 13 (1), 1-9
- **AMBACH A** ; **BONNEKOH B** ; **NGUYEN M et al.** Imiquimod, a Toll-like Receptor-7 Agonist, Induces Perforin in Cytotoxic T Lymphocytes in Vitro. *Mol Immunol*, 2004, vol. 40, 1307-1314
- The Science and Practice of Pharmacy. Mack Publishing Company, 1995
- *J. Med. Chem*, 2005, vol. 48, 3481-3491